(19) **Europäisches Patentamt**
**European Patent Office**
**Office européen des brevets**

(11) **EP 1 478 659 B1**

(12) **EUROPEAN PATENT SPECIFICATION**

(45) Date of publication and mention
of the grant of the patent:
**12.11.2008 Bulletin 2008/46**

(21) Application number: **02749008.5**

(22) Date of filing: **11.07.2002**

(51) Int Cl.:
*C07K 7/06* (2006.01)  *C07K 7/08* (2006.01)
*C07K 5/107* (2006.01)  *C07K 5/117* (2006.01)
*C07K 5/087* (2006.01)  *C07K 5/083* (2006.01)
*A61K 38/04* (2006.01)  *C07K 14/805* (2006.01)
*A61K 38/42* (2006.01)  *C07K 14/47* (2006.01)
*A61K 38/17* (2006.01)  *A61P 9/10* (2006.01)
*A61P 37/02* (2006.01)  *A61P 35/00* (2006.01)
*A61P 31/20* (2006.01)  *A61P 3/04* (2006.01)

(86) International application number:
**PCT/GB2002/003203**

(87) International publication number:
**WO 2003/006492 (23.01.2003 Gazette 2003/04)**

(54) **BIOLOGICALLY ACTIVE PEPTIDES**

BIOLOGISCH AKTIVE PEPTIDE

PEPTIDES EXERÇANT UNE ACTIVITE BIOLOGIQUE

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **13.07.2001 US 904492**

(43) Date of publication of application:
**24.11.2004 Bulletin 2004/48**

(60) Divisional application:
**07000950.1 / 1 790 655**
**08005150.1 / 1 947 109**

(73) Proprietor: **CMS Peptides Patent Holding Company Limited**
**Trident Chambers**
**P.O.Box 146**
**Road Town**
**Tortola (VG)**

(72) Inventors:
• **WONG, Wai, Ming**
  **Hong Kong (CN)**
• **LAM, Kong**
  **Nanshan District,**
  **518053 Shenzhen (CN)**

(74) Representative: **Baldock, Sharon Claire et al**
**BOULT WADE TENNANT**
**Verulam Gardens**
**70 Gray's Inn Road**
**London WC1X 8BT (GB)**

(56) References cited:
**WO-A-00/63233**      **WO-A-91/09613**
**WO-A-96/18646**      **WO-A-97/35602**
**WO-A-98/12219**      **FR-A- 2 794 370**
**US-A- 5 556 744**    **US-A- 5 861 483**

• **FOGACA E.A.: "Antimicrobial activity of bovine hemoglobin fragment in the tick Boophilus micrplus" JOURNAL OF BIOLOGICAL CHEMISTRY, vol. 274, no. 36, 1999, pages 25330-25334, XP002236974 MD US**
• **KARELIN A A ET AL: "PROTEOLYTIC DEGRADATION OF HEMOGLOBIN IN ERYTHROCYTES LEADS TO BIOLOGICALLY ACTIVE PEPTIDES" PEPTIDES, ELMSFORD, US, vol. 16, no. 4, 1995, pages 693-697, XP000983865 ISSN: 0196-9781**

- SCHALLY E.A.: "Isolation, structural elucidation and synthesis of a tetradecapeptide with in vitro ACTH-releasing activity corresponding to residues 33-46of the alpha-chain of porcine hemoglobin" BIOCHEMICAL AND BIOPHYSICAL RESEARCH COMMUNICATIONS, vol. 82, no. 2, 1978, pages 582-588, XP008015843 ORLANDO, FL US
- CHANG R C C ET AL: "ISOLATION AND STRUCTURE OF SEVERAL PEPTIDES FROM PROCINE HYPOTHALAMI" BIOCHIMICA ET BIOPHYSICA ACTA, AMSTERDAM, NL, vol. 625, 1980, pages 266-273, XP002905222 ISSN: 0006-3002
- KARELIN A A ET AL: "[Proteolytic degradation of hemoglobin in erythrocytes results in formation of biologically active peptides]" BIOORGANICHESKAIA KHIMIIA. RUSSIA APR 1998, vol. 24, no. 4, April 1998 (1998-04), pages 271-281, XP008015845 ISSN: 0132-3423
- LADRAM ET AL: "Characterization of receptors for thyrotropin-releasing hormone-receptors potentiating peptide on rat anterior pituitary membranes" JOURNAL OF BIOLOGICAL CHEMISTRY, AMERICAN SOCIETY OF BIOLOGICAL CHEMISTS, BALTIMORE, MD, US, vol. 267, no. 36, 25 December 1997 (1997-12-25), pages 25697-26702, XP002149258 ISSN: 0021-9258
- SQUIRE E.A.: "Leucine aminopeptidase-like activity in Aplysia hemolymph rapidly degrades biologically active alpha-bag cell peptide fragments" JOURNAL OF BIOLOGICAL CHEMISTRY, vol. 266, no. 33, 25 November 1991 (1991-11-25), pages 22355-22363, XP002245007 MD US
- FURKA A ET AL: "STRING SYNTHESIS A SPATIALLY ADDRESSABLE SPLIT PROCEDURE" JOURNAL OF COMBINATORIAL CHEMISTRY, AMERICAN CHEMICAL SOCIETY, WASHINGTON, US, vol. 2, no. 3, 19 May 2000 (2000-05-19), pages 220-223, XP001058980 ISSN: 1520-4766
- BULANT M ET AL: "PROCESSING OF THYROTROPIN-RELEASING HORMONE PROHORMONE(PRO-TRH) GENERATES A BIOLOGICALLY ACTIVE PEPTIDE,PREPRO-TRH-(160-169), WHICH REGULATES TRH-INDUCED THYROTROPIN SECRETION" PROCEEDINGS OF THE NATIONAL ACADEMY OF SCIENCES OF USA, NATIONAL ACADEMY OF SCIENCE. WASHINGTON, US, vol. 87, no. 12, 1 June 1990 (1990-06-01), pages 4439-4443, XP000134334 ISSN: 0027-8424
- PATI E.A.: ENDOCRINOLOGY, vol. 136, no. 1, 1995, pages 75-84,

SEQUENCE LISTING

<110> Wong, Wai Ming
Lin, Gang

<120> BIOLOGICALLY ACTIVE PEPTIDES

<130> WILKG3.001CP1

<140> unknown
<141> 2002-06-20

<150> 09/904,492
<151> 2001-07-13

<160> 30

<170> FastSEQ for Windows Version 4.0

<210> 1
<211> 10
<212> PRT
<213> Sus scrofa

<400> 1

```
Pro Thr Thr Lys Thr Tyr Phe Pro His Phe
1               5               10
```

<210> 2
<211> 9
<212> PRT

<213> Sus scrofa

<400> 2

Val Val Tyr Pro Trp Thr Gln Arg Phe
1               5

<210> 3
<211> 13
<212> PRT
<213> Sus scrofa

<400> 3

Lys Ala Val Gly His Leu Asp Asp Leu Pro Gly Ala Leu
1               5                   10

<210> 4
<211> 18
<212> PRT
<213> Sus scrofa

<400> 4

Val Ala Pro Glu Glu His Pro Thr Leu Leu Thr Glu Ala Pro Leu Asn
1               5                   10                  15
Pro Lys

<210> 5
<211> 13
<212> PRT
<213> Sus scrofa

<400> 5

Leu Gly Met Glu Ala Cys Gly Ile His Glu Thr Thr Tyr
1               5                   10

<210> 6
<211> 11
<212> PRT
<213> Sus scrofa

<400> 6

Leu Arg Val Ala Pro Glu Glu His Pro Val Leu
1               5                   10

<210> 7
<211> 12
<212> PRT
<213> Sus scrofa

<400> 7

Ala Ala His His Pro Asp Asp Phe Asn Pro Ser Val
1                   5                   10


<210> 8
<211> 13
<212> PRT
<213> Sus scrofa

<400> 8

Pro Ser Ile Val Gly Arg Pro Arg His Gln Gly Val Met
1                   5                   10


<210> 9
<211> 13
<212> PRT
<213> Sus scrofa

<400> 9

Ile Gly Met Glu Ser Ala Gly Ile His Glu Thr Thr Tyr
1                   5                   10


<210> 10
<211> 9
<212> PRT
<213> Sus scrofa

<400> 10

Val Gly Met Gly Glu Lys Asp Ser Tyr
1                   5


<210> 11
<211> 9
<212> PRT
<213> Sus scrofa

<400> 11

Val Gly Met Gly Gln Lys Asp Ser Tyr
1                   5

<210> 12
<211> 10
<212> PRT
<213> Sus scrofa

<400> 12

```
Val Gly Met Gly Gln Lys Asp Ser Tyr Val
1               5                   10
```

<210> 13
<211> 10
<212> PRT
<213> Sus scrofa

<400> 13

```
Met Ala Thr Ala Ala Ser Ser Ser Ser Leu
1               5                   10
```

<210> 14
<211> 3
<212> PRT
<213>Sus scrofa

<400> 14

```
Tyr Ser Phe
1
```

<210> 15
<211> 3
<212> PRT
<213> Sus scrofa

<400> 15

```
Ala Ala Phe
1
```

<210> 16
<211> 3
<212> PRT
<213> Sus scrofa

<400> 16

```
Tyr Ser Leu
1
```

<210> 17

<211> 7
<212> PRT
<213> Sus scrofa

<400> 17

Thr Thr Tyr Asn Ser Ile Met
1               5

<210> 18
<211> 5
<212> PRT
<213> Sus scrofa

<400> 18

Phe Glu Glu Asn Met
1               5

<210> 19
<211> 5
<212> PRT
<213> Sus scrofa

<400> 19

Phe Glu Pro Ser Phe
1               5

<210> 20
<211> 4
<212> PRT
<213> Sus scrofa

<400> 20

Phe Asn Glu Glu
1

<210> 21
<211> 4
<212> PRT
<213> Sus scrofa

<400> 21

Phe Glu Glu Met
1

<210> 22
<211> 4
<212> PRT

<213> Sus scrofa

<400> 22

```
Phe Glu Glu Glu
1
```

<210> 23
<211> 4
<212> PRT
<213> Sus scrofa

<400> 23

```
Phe Glu Ser Phe
1
```

<210> 24
<211> 4
<212> PRT
<213> Sus scrofa

<400> 24

```
Pro Glu Asn Phe
1
```

<210> 25
<211> 4
<212> PRT
<213> Sus scrofa

<400> 25

```
Phe Val Asn Asp
1
```

<210> 26
<211> 5
<212> PRT
<213> Sus scrofa

<400> 26

```
Phe Gln Pro Ser Phe
1               5
```

<210> 27
<211> 6
<212> PRT

<213> Sus scrofa

<400> 27

```
Phe Asn Phe Val Pro Pro
1               5
```

<210> 28
<211> 10
<212> PRT
<213> Sus scrofa

<400> 28

```
Ala Gly Asp Asp Ala Pro Arg Ala Val Phe
1               5               10
```

<210> 29
<211> 11
<212> PRT
<213> Sus scrofa

<400> 29

```
Leu Arg Val Ala Pro Glu Glu His Pro Thr Leu
1               5               10
```

<210> 30
<211> 10
<212> PRT
<213> Sus scrofa

<400> 30

```
Arg Val Ala Pro Glu Glu His Pro Thr Leu
1               5               10
```

**Description**

**FIELD OF INVENTION**

The present invention is related to the field of immunology. In particular, the present invention is directed to peptides and their pharmaceutical compositions which are capable of modulating immune responses.

**BACKGROUND OF INVENTION**

Peptides are known in the art for treatment of diseases and as pharmaceutical compositions. For example, US Patent No.6,191,113 discloses a peptide that has inhibitory activity for the growth of smooth muscle cells and is therefore useful for preventing and treating pathological conditions associated with growth of smooth muscle cells such as arteriosclerosis, restenosis after angioplasty, luminal stenosis after grafting blood vessel and smooth muscle sarcoma. US 6,184,208 discloses another peptide that is found to modulate physiological processes such as weight gain activity of the epithelial growth zone and hair growth.

Peptides are also known to carry other biological functions, such as neurotransmitter activity. For example, a neutrotransmitter $\alpha$-bag cell peptide (V-BCP(1-9)), which is released by the bag cells in the abdominal ganglion of the marine snail *Apysia*, is described by Squire and coworkers (Squire et al., J. Biol. Chem, vol. 266, no. 33, 1991, p. 22366-22363).

Many methods to synthesize and screen peptides are present. For example, a method to prepare combinatorial peptide libraries had been suggested by Furka and coworkers (Furka et al., J. Comb. Chem.. 2000, 2, 220-223). Nonetheless, the pharmaceutical functions of these disclosed peptides and many other undisclosed peptides remain unknown.

**SUMMARY OF INVENTION**

It is therefore an object of the present invention to identify biologically active polypeptides. Many peptides were synthesized by standard chemical methods and screened for their biological activity. The peptides are given codes having the letters CMS followed by a number. A total of 30 peptides have been identified as having in vivo biological activities. The sequences and the corresponding ID numbers of these biologically active peptides are shown in Table A.

Table A

| Sequence Listing ID No. | Peptide Name | Peptide Sequence |
|---|---|---|
| 1 | CMS001 | Pro Thr Thr Lys Thr Tyr Phe Pro His Phe |
| 2 | CMS002 | Val Val Tyr Pro Trp Thr Gln Arg Phe |
| 3 | CMS008 | Lys Ala Val Gly His Leu Asp Asp Leu Pro Gly Ala Leu |
| 4 | CMS010 | Val Ala Pro Glu Glu His Pro Thr Leu Leu Thr Glu Ala Pro Leu Asn Pro Lys |
| 5 | CMS012 | Leu Gly Met Glu Ala Cys Gly Ile His Glu Thr Thr Tyr |
| 6 | CMS013 | Leu Arg Val Ala Pro Glu Glu His Pro Val Leu |
| 7 | CMS014 | Ala Ala His His Pro Asp Asp Phe Asn Pro Ser Val |
| 8 | CMS015 | Pro Ser Ile Val Gly Arg Pro Arg His Gln Gly Val Met |
| 9 | CMS016 | Ile Gly Met Glu Ser Ala Gly Ile His Glu Thr Thr Tyr |
| 10 | CMS018 | Val Gly Met Gly Glu Lys Asp Ser Tyr |
| 11 | CMS019 | Val Gly Met Gly Gln Lys Asp Ser Tyr |
| 12 | CMS020 | Val Gly Met Gly Gln Lys Asp Ser Tyr Val |
| 13 | CMS021 | Met Ala Thr Ala Ala Ser Ser Ser Ser Leu |
| 14 | CMS022 | Tyr Ser Phe |
| 15 | CMS023 | Ala Ala Phe |
| 16 | CMS024 | Tyr Ser Leu |

EP 1 478 659 B1

(continued)

| Sequence Listing ID No. | Peptide Name | Peptide Sequence |
|---|---|---|
| 17 | CMS026 | Thr Thr Tyr Asn Ser Ile Met |
| 18 | CMS027 | Phe Glu Glu Asn Met |
| 19 | CMS028 | Phe Glu Pro Ser Phe |
| 20 | CMS029 | Phe Asn Glu Glu |
| 21 | CMS030 | Phe Glu Glu Met |
| 22 | CMS032 | Phe Glu Glu Glu |
| 23 | CMS033 | Phe Glu Ser Phe |
| 24 | CMS034 | Pro Glu Asn Phe |
| 25 | CMS035 | Phe Val Asn Asp |
| 26 | CMS036 | Phe Gln Pro Ser Phe |
| 27 | CMS003 | Phe Asn Phe Val Pro Pro |
| 28 | CMS007 | Ala Gly Asp Asp Ala Pro Arg Ala Val Phe |
| 29 | CMS009 | Leu Arg Val Ala Pro Glu Glu His Pro Thr Leu |
| 30 | CMS011 | Arg Val Ala Pro Glu Glu His Pro Thr Leu |

Accordingly, described herein are peptides having sequences identified as sequence ID No.1 to sequence ID No.30.

Additionally described herein are nucleic acids that have sequences co g for the peptides identified above as sequence ID No.1 to 30 and expression vectors that contain the nucleic acid sequences of the peptides shown below as sequence ID No.1 to 30.

Also described herein are hybrid peptides containing a leader or signal peptide adjacent a peptide, the peptide comprising an amino acid sequence selected from the group consisting of SEQ ID NOs. 1-30 or a functional derivative of a peptide having an amino acid sequence selected from the group consisting of SEQ ID NOs. 1-30.

Also described is a genetic vector comprising a nucleotide sequence encoding a peptide comprising a leader amino acid sequence adjacent a peptide selected from the group consisting of SEQ ID NOs. 1-30.

The peptides produced in any of the above-described genetic vectors can modulate, but not limited to modulating, one or more of the following : immune activity; hepatitis infection, including but not limited to hepatitis B infection; nephritis; the growth of a cancer, including but not limited to sarcoma, liver cancer, leukemia and melanoma; and body weight.

Further described is a micro-organism with a genome comprising a nucleotide sequence encoding a peptide comprising an amino acid sequence selected from the group consisting of SEQ ID NOs. 1-30.

Exogenous peptide as used herein refers to a peptide having an amino acid sequence that is different from any other peptides normally expressed the micro-organism in its natural, unmodified form.

Further described is a micro-organism with a genetic composition comprising a nucleotide sequence encoding an exogenous hybrid peptide comprising a leader amino acid sequence adjacent a peptide, the peptide comprising an amino acid sequence selected from the group consisting of SEQ ID NOs. 1-30.

Also described is a micro-organism with a genetic composition comprising a nucleotide sequence encoding an exogenous hybrid peptide comprising a leader amino acid sequence adjacent a peptide, selected from the group consisting of SEQ ID NOs. 1-30.

The peptides produced in any of the above-described micro-organism can modulate, but not limited to modulating, one or more of the following : immune activity; hepatitis infection, including but not limited to hepatitis B infection; nephritis; the growth of a cancer, including but not limited to sarcoma, liver cancer, leukemia and melanoma; and body weight.

A first aspect of the present invention relates to a pharmaceutical composition comprising a peptide consisting of an amino acid sequence YSL (SEQ ID NO. 16).

In a specific embodiment, the peptides present in the above-described pharmaceutical composition can modulate, but is not limited to modulating, one or more of the following : immune activity and the growth of a cancer, including but not limited to liver cancer and melanoma.

4

Yet a further aspect of the present invention relates to a method of making a pharmaceutical composition comprising providing a peptide consisting of an amino acid sequence of SEQ ID NO. 16 and mixing said peptide with a pharmaceutically acceptable carrier.

In connection with the above-described method, the peptide can modulate, but not limited to modulating, one or more of the following : immune activity and the growth of a cancer, including but not limited to liver cancer, and melanoma.

Yet a further aspect of the present invention relates to a peptide consisting of an amino acid sequence of SEQ ID NO 16 for use in the treatment of disease.

In a specific embodiment, the peptide used for the treatment of disease described above may be used to modulate, but not limited to modulating, one or more of the following human conditions: immune activity, and the growth or a cancer, including but not limited to liver cancer and melanoma.

## DETAILED DESCRIPTION

The peptides can bye readily synthesized by standard synthetic methods from L-amino acids, but may also be synthesized by genetic engineering methods using nucleic acids that have sequences encoding the individual peptides.

### I. Biological Activity

In order to investigate the possible biological activity of peptides, the immunological effect of the peptides on animal model was examined, with procedures compliant to the "Principles of Pre-clinical Research of New Drugs" issued by Ministry of Health of People's Republic of China [1].

The T lymphocyte transformation test, NK cell cytotoxicity activity test, and the T lymphocyte IL-2 and IFN-$\gamma$ secretion test were used to detect any possible effect of peptides on specific cellular immune function. The carbon particle clearance test was used to detect any possible effect of peptides on nonspecific cellular immune function. The Sheep Red Blood Cell (SRBC) hemolysis test was used to detect any possible effect of peptides on humoral immune function. The immunity organ weight test was used to detect any possible effect of peptides at the organ level.

In this study, the saline group was used as negative control, while the IL-2 and IFN-$\gamma$ groups were used as positive controls, since IL-2 and IFN-$\gamma$ are well-studied immunostimulants [10]. Four arbitrary concentrations of sample peptides were used in this study, to cover a 1000 fold dosage range. Due to the intrinsic complexity of in vivo immunological response, and the lack of prior knowledge on the dosage versus response function, therefore any statistically significant difference over the negative control in any of the dosage groups has been scored as positive biological activity.

Results of this study were as follows:

1. Peptides CMS001, CMS002, CMS003, CMS007, CMS008, CMS009, CMS010, CMS011, CMS012, CMS015, CMS019, CMS021, CMS029, and CMS034 were found to be able to enhance T lymphocyte transformation, having statistically significant difference from the saline normal control group. Peptides CMS014 and CMS036 were also found to be able to inhibit T lymphocyte transformation, having statistically significant difference from the saline normal control group.

2. Peptides CMS001, CMS002, CMS003, CMS008, CMS009, CMS010, CMS011, CMS012, CMS013, CMS015, CMS016, CMS020, CMS021, CMS022, CMS023, CMS024, CMS026, CMS027, CMS028, CMS029, CMS030, CMS032, CMS033, CMS034, CMS035, and CMS036 were found to be able to increase the cytotoxic activity of NK cells, having statistically significant difference from the saline normal control group. Peptides CMS008 and CMS012, at suitable concentration, were also found to be able to decrease the cytotoxic activity of NK cells, having statistically significant difference from the saline normal control group.

3. Peptides CMS001, CMS003, CMS007, CMS009, CMS010, CMS011, CMS012, CMS015, CMS020, CMS022, and CMS034 were found to be able to enhance the secretion of interleukin-2 (IL-2) by T lymphocytes, having statistically significant difference from the saline normal control group.

4. Peptides CMS001', CMS003, CMS009, CMS010, CMS011, CMS012, CMS013, CMS016, CMS021, CMS022, and CMS028 were found to be able to enhance the secretion of IFN by T lymphocytes, having statistically significant difference form the saline normal control group.

5. Peptides CMS001, CMS002, CMS003, CMS007, CMS008, CMS009, CMS010, CMS011, CMS012, CMS013, CMS014, CMS015, CMS016, CMS018, CMS019, CMS020, CMS021, CMS022, CMS023, CMS024, CMS026, CMS027, CMS028, CMS029, CMS030, CMS032, CMS033, CMS034, CMS035, and CMS036 were found to be able to enhance the synthesis of anti-SRBC antibody upon the antigenic challenge, having statistically significant difference from the saline normal control group. Peptides CMS002, CMS003, CMS009, CMS010, CMS011, CMS013, CMS014, CMS015, CMS018, CMS019, CMS020, CMS026, CMS028, CMS029, CMS030, CMS034, and CMS036, at suitable concentration, were also found to be able to inhibit the synthesis of anti-SRBC antibody upon the antigenic challenge, having statistically significant difference from the saline normal control group.

6. Peptides CMS003, CMS008, CMS009, CMS010, CMS011, CMS013, CMS016, CMS018, CMS019; CMS020, CMS022, CMS024, CMS027, CMS030, CMS035, CMS036 were found to be able to enhance the phagocytotic activity of mononuclear phagocyte, having statistic significant difference from the saline normal control group.

7. Peptides CMS001, CMS002, CMS008, CMS010, CMS012, CMS013, CMS014, CMS015, CMS016, CMS018, CMS019, CMS020, CMS021, CMS022, CMS023, CMS024, CMS026, CMS027, CMS028, CMS029, CMS030, CMS032, CMS033, CMS034, CMS035, and CMS036 were found to be able to increase the weight of the thymus gland, having statistically significant difference from the saline normal control group.

8. Peptides CMS019, CMS020, and CMS030 were found to be able to increase the weight of the spleen, having statistically significant difference from the saline normal control group. Peptides CMS001, CMS003, CMS007, CMS008, CMS009, CMS010, CMS011, CMS013, CMS014, CMS015, CMS021, CMS023, CMS024, CMS027, CMS029, and CMS036, at suitable concentration, were also found to be able to decrease the weight of the spleen, having statistically significant difference from the saline normal control group.

The materials and methods used to analyse the effect of the peptides on mouse are described below:

Materials

1. Experimental Animal

BALB/c Mice, 18-22g weight, 50% female and 50% male, provided by Experimental Animal Center, National Institute of Medical Science, PR China.

2. Administration

recombinant mouse IFN-$\gamma$ (rmIFN-$\gamma$) group: $3 \times 10^5$ IU/kg/day
recombinant human IL (rnIL)-2 group: $3 \times 10^5$ IU/kg/day
Saline group: 0.5ml/each/day
peptide dose I group: 500$\mu$g/kg/day
peptide dose II group: 50$\mu$g/kg/day
peptide dose III group: 5$\mu$g/kg/day
peptide dose IV group: 0.5$\mu$g/kg/day
The above substances were all dissolved in 0.5ml saline and injected intraperitoneal (i.p.) for 15 continuous days, once per day.

3. Main Reagents

The peptides were custom manufactured by American Peptide Company, Inc., USA
Fetal bovine serum, and RPMI-1640 cell culture medium, Gibco, USA
MTT, and ConA, Sigma, USA
rmIFN-$\gamma$, Beijing Biotech Inc., China
rhIL-2, Shanghai Huaxin Biotech Inc., China
Lymphocyte separation solution, Research Institute of Hematologic Disease, National Institute of Medical Science, PR China
Vesicular Stomatitis Virus (VSV), IFN-$\gamma$ and IL-2 standard sample, National Institute For The Control Of Pharmaceutical And Biological Products, PR China HT-2 cell and L929 cell, gift from Prof WF Chen of Beijing University Department of Immunology, PR China

Method

1. The effect of peptides on cellular immunity

1.1 Preparation of spleen cell suspension [1,2]

The BALB/c mice were randomly divided into the peptide, IFN, IL-2, and saline groups. Ten mice per group. The day after the last test substance administration, the mice were sacrificed by cervical dislocation. The spleen was isolated aseptically and manually dispersed in cold D-Hank's solution using an injection needle. The dispersed cell suspension was further sieved through a 100 gauge 150$\mu$m diameter stainless steel sieve. After centrifugation at 200g for 10 minutes, the supernatant was discarded. The cell pellet was re-suspended in 10 volume of Tris-NH$_4$Cl buffer and then kept standing still for 10 minutes at room temperature. The suspended cells were collected by centrifugation at 150g for 10 minutes. The cells were washed 2-4 times with cold D-Hank's solution by re-suspending and collecting by centrifugation with condition as described above. The washed cells were then diluted to the desired cell densities by RPMI-1640 culture medium, containing 10% fetal bovine serum.

1.2 The effect of peptides on T lymphocyte transformation[1,2]

Spleen cells of density $1\times10^6$/ml were placed onto a 96 wells cell culture plate, 100$\mu$l/well, three parallel wells each of the assay sample and control sample per mouse. To the assay wells, 100$\mu$l/well ConA of 100$\mu$g/ml in RPMI-1640 was added, and 100$\mu$l/well plain RPMI-1640 was used for the controls. The cells were incubated for 66 hrs at 37°C, 5% CO$_2$. Then cells were then pelleted by centrifugation at 150g for 10 minutes. The supernatant was collected and stored at -20°C for cytokines IL-2 and IFN determination.

50$\mu$l/well MTT of 1mg/ml in RPMI-1640 was added to the cell pellet and the cells re-suspended by shaking for 2 minutes. The incubation was continued for 4 hours. The supernatant was discarded after centrifugation at 150g for 10 minutes. 120$\mu$l 40mM HCl-2-propanol was added to the cell pellet and shaken for 3 minutes. to obtain OD$_{570}$nm of each well referenced at 630nm. An ELISA reader was used

Calculation:

Each mouse formed three assay wells and three control wells. The Stimulation Index (SI) of each mouse was obtained by first deriving the average OD of the three parallel wells, then dividing the value of the assay wells by the control wells.

1.3 The effect of peptides on NK cell activity[3,4]

Mice spleen cells were prepared to $4\times10^6$/ml as described in section 1.1 above. Target cells YAC-1 were brought to log phase and adjusted to $1\times10^5$/ml. Using a 96 wells cell culture plate, 100$\mu$l mouse spleen cells and 100$\mu$l culture medium were added to the control well containing only the spleen cells; 100$\mu$l target cells and 100$\mu$l culture medium were added to the control well containing only target cells; 100$\mu$l mouse spleen cells and 100$\mu$l target cells were added to the NK activity assay well. Three parallel sets of the above were prepared per mouse.

Samples were centrifuged at 150g for 10 minutes to collect the cells. The supernatant was discarded and 50$\mu$l/well MTT of 1mg/ml was added. The reactions mixture was then shaken for 2 minutes, and incubated at 37°C, 5% CO$_2$ for 4 hours. The supernatant was discarded after centrifugation at 150g for 10 minutes. 120 $\mu$l 40mM HCl-2-propanol was added and shaken for 3 minutes. An ELISA reader was used to obtain OD$_{570}$nm of each well referenced at 630nm.

Calculation:

Each mouse has 9 wells: three "spleen cells only control, three target cells only control, and three assay wells with both spleen and target cells. The NK cell activity index of each mouse was obtained by first deriving the average OD of the three parallel wells of each combination, then applying this average OD to the following formula:

$$\text{NK cell activity index} = [1-(\text{average OD of spleen and target cell well} - \text{average OD of spleen cell only well}) \div (\text{average OD of target cell only well})] \times 100\%$$

1.4 The effect of peptides on the activity T lymphocyte in secreting IL-2[5]

HT-2 cells at log phase were collected by centrifugation at 150g for 10 minutes, and washed three times with cold Hank's solution by re-suspension and centrifugation. The collected HT-2 cells were.re-suspended in RPMI-1640 and incubated at 37°C, 5% $CO_2$ for 30 minutes. The cells were further washed twice with RPMI-1640 by re-suspension and centrifugation, and re-suspended to final concentration of $2 \times 10^5$/ml. with RPMI-1640.

The supernatant obtained in section 1.2 were diluted to the following percentage with RPMI-1640: 100%, 50%, 25%, 12.5%, 6.25%, and 3.125%.

rIL-2 was diluted to the following concentration with RPMI-1640: 500IU/ml, 250IU/ml, 125IU/ml, 62.5IU/ml, 31.25IU/ml, and 15.5IU/ml.

A 96 well cell culture plate was set up with three parallel wells per combination:

Negative control: 100μl RPMI-1640 + 100μl HT-2 cell suspension

rIL-2 standard: 100μl rIL-2 solution + 100μl HT-2 cell suspension

Assay well: 100μl diluted supernatant + 100μl HT-2 cell suspension

The plate was incubated at 37°C, 5% $CO_2$ for 68 hours, then centrifuged at 150g for 15 minutes and the supernatant removed. 100μl 0.5mg/ml MTT. in RPMI-1640 without phenolsulfonphthalein was added into each well. After shaking for 3-4 minutes to re-suspend the cells, continue to incubate for another 4 hours. Samples were then centrifuged at 150g for 15 minutes and the supernatant was removed. To each well, 120μl 40mM HCl-2-propanol was added, mixed for 3-4 minutes and OD analyzed at 570nm, referenced at 630nm with an ELISA plate reader.

Calculation:

The average OD of the three parallel wells of each dilution was taken and plotted against concentration on a semi-log paper, concentration on the X-axis. The concentration at 50% OD saturation was obtained for both the testing supernatant and rIL-2.

Sample IL-2 activity = (sample dilution at 50% maximum action ÷ rIL-2 standard dilution at 50% maximum action) x activity of rIL-2 standard at 50% maximum action (IU/ml)

1.5 The effect of peptides on the activity of T lymphocyte in secreting interferon (IFN)[6]

The supernatant from the section 1.2 was diluted with RPMI-1640 culture medium to the following percentages: 100%, 50%, 25%, 12.5%, 6.25%, and 3.125%.

The recombinant interferon (rIFN) standard was diluted with RPMI-1640 to the following concentrations: 500IU/ml, 250IU/ml, 125IU/ml, 62.5IU/ml, 31.25IU/ml, and 15.5IU/ml.

Target cells L929 at log phase were adjusted to $2 \times 10^5$/ml with RPMI-1640, with treatment same as the HT-2 cell described in section 1.4. Stock VSV was also adjusted to 100 $TCID_{50}$ with RPMI-1640.

The following on a 96 well culture plate was set up, three parallel wells per combination:

Negative control well: 150μl RPMI-1640 + 100μl L929

Positive control well: 100μl RPMI-1640 + 100μl L929 + 50μl VSV

rIFN activity well: 100μl rIFN standard + 100μl L929 + 50μl VSV

assay well: 100μl diluted supernatant + 100μl L929 + 50μl VSV

Samples were incubated at 37°C, 5% $CO_2$ for 24 hours. The positive control wells were observed periodically under inverted microscope to confirm cell lysis, then collected, washed, and OD of all wells was read same as described in section 1.4.

Calculation:

Concentrations at 50% maximum action were obtained same way as section 1.4. Calculate IFN activity of sample as following:

$$\text{Sample IFN activity} = (\text{sample dilution at 50\% maximum action} \div \text{rIFN standard dilution at 50\% maximum action}) \times \text{standard rIFN activity at that 50\% maximum action (IU/ml)}$$

2. The effect of peptides on antibody formation[7]

Sheep red blood cells (SRBC) were prepared by collecting blood from cervical vein and put into a sterile flask with glass beads. The flask was shaken for 3 minutes and the blood then mixed with Alsever solution (glucose 2.05g, NaCl 0.4g, Na lemonade 0.8g, adjust to 100ml with distilled water) and stored at 4°C. Immediately before use, samples were centrifuged at 130g, 5 minutes to collect the SRBC. The cells were washed two times by re-suspension and centrifugation in normal saline. Then the cell pellet was collected by centrifugation at 180g for 10 minutes and re-suspended in saline to make the final working SRBC suspension, 2% (v/v).

Complement was prepared by adding 10 volumes of fresh Cavy serum into one volume centrifuge packed SRBC, and then gently shaking for 30 minutes at 4°C. The SRBC was removed by centrifugation at 200g for 10 minutes. 10 volumes of normal saline were added to obtain the working complement solution.

The BALB/c mice were randomly divided into the peptide group, IFN group, IL-2 group, and saline group, 10 mice per group. The test substances were administered as described in section 1.1, plus intraperitoneal injection 0.2ml SRBC per mouse on day 12. On the day after the last test substance administration (day 16), blood was collected from the eye canthus and left at room temperature for one hour for serum exudation. After centrifugation at 200g for 10 minutes, the serum was diluted by 500 times with normal saline.

To 1ml diluted mouse serum of each mouse, 0.5ml SRBC suspension was added. Ice cold. Then 1ml working complement solution was added and incubated at 37°C water bath for 10 minutes. Reactions were terminated by ice cold. Samples were then centrifuged at 200g for 10 minutes to obtain the supernatant.

To 1ml of this supernatant, 3ml Drabkin solution was added and left at room temperature for 10 minutes. $OD_{540nm}$ was obtained.

Calculation:

Reference $OD_{540nm}$ was obtained by mixing 0.25ml SRBC suspension with Drabkin solution to 4ml and placed at room temperature for 10 minutes before $OD_{540nm}$ was taken.

$$\text{Sample serum index} = (OD_{540nm} \text{ of test sample} \div \text{reference } OD_{540nm}) \times 500$$

3. The effect of peptides on the phagocytosis function of mononuclear phagocyte and the weight of immune organ[8,9].

On the next day after the last test substance administration (day 16), the mice were injected with 0.1ml/kg body weight India ink (5 times dilution with normal saline) from the tail vein.

One minute and five minutes after Indian ink injection, 20μl blood was obtained from the eye canthus with a heparinized tubing. The blood was mixed with 2ml 0.1 % w/v $Na_2CO_3$ and then $OD_{680nm}$ obtained. The outline clear index K was calculated by the following formula:

$$K = (\lg A_1 - \lg A_2) \div (t2 - t1)$$

Key:

A1: OD680nm at first minute
A2: OD680nm at fifth minute
t2: 5 minutes
t2: 1 minute

One day after the last test substance administration (day 16), the liver, spleen, and thymus gland were separated and blotted dry with filter paper and weighed. The phagocytosis index α was calculated as below:

$$\alpha = (^3 \sqrt{K})(W \div W_{LS})$$

key:

W: body weight
$W_{LS}$: weight of liver plus spleen
Thymus gland index (%) = (thymus weight / body weight) $\times$ 100%
Spleen index (%) = (spleen weight / body weight) $\times$ 100%

Results

Due to the large quantity of raw data involved, only the compiled end result are presented. The groups without statistically significant difference from the saline negative control are also omitted.

1. The effect of peptides on T lymphocyte transformation

At 500μg/kg/day, CMS002, CMS007, CMS008, CMS010, CMS012, CMS015, CMS019, CMS021, and CMS029 were found to be able to enhance T lymphocyte transformation, having statistically significant difference from the saline group (P<0.05). Among these peptides, CMS010 and CMS015 were found to have statistically significant difference from the IFN-γ group and IL-2 group (P<0.05) as shown in Table 1 below.

Table 1

| Group | N | X±SD (stimulation index) |
|---|---|---|
| CMS002 | 8 | 1.8±0.3* |
| CMS007 | 9 | 1.6±0.1* |
| CMS008 | 9 | 1.7±0.1* |
| CMS009 | 10 | 1.7±0.2* |
| CMS010 | 9 | 2.0±0.3*@^ |
| CMS012 | 9. | 1.6±0.2* |
| CMS015 | 9 | 1.9±0.3*@^ |
| CMS019 | 9 | 1.8±0.3* |
| CMS021 | 10 | 1.6±0.1* |
| CMS029 | 9 | 1.7±0.3* |
| IFN-γ | 10 | 1.6±0.2* |
| IL-2 | 10 | 1.7±0.2* |
| Saline | 10 | 1.3±0.1 |

* comparing to saline group P<0.05
@ comparing to IFN-γ group P<0.05
^ comparing to IL-2 group P<0.05

At 50μg/kg/day, CMS001, CMS002 and CMS003 were found to be able to stimulate T lymphocyte transformation, having statistically significant difference from saline group, IFN-γ group, and IL-2 group (P<0.05). CMS014 and CMS036 were found to be able to inhibit T lymphocyte transformation, having statistical significant difference from saline group (P<0.05). Data detailed in Table 2 below.

Table 2

| Group | N | X±SD (stimulation index) |
|---|---|---|
| CMS001 | 10 | 2.2±0.5*@^ |
| CMS002 | 10 | 2.6±0.3*@^ |
| CMS003 | 8 | 2.2±0.5*@^ |
| CMS014 | 9 | 1.0±0.1* |
| CMS036 | 9 | 1.0±0.1* |

| | | (continued) | |
|---|---|---|---|
| Group | N | X±SD (stimulation index) | |
| IFN-γ | 9 | 1.7±0.2* | |
| IL-2 | 10 | 1.8±0.2* | |
| Saline | 10 | 1.3±0.1 | |
| * comparing to saline group P<0.05 @ comparing to IFN-γ group P<0.05 ^ comparing to IL-2 group P<0.05 | | | |

At 5/μg/kg/day, CMS001, CMS003, CMS007, and CMS034 were found to be able to stimulate T lymphocyte transformation, having statistically significant difference from the saline group (P<0.05) as shown in Table 3.

Table 3

| Group | N | X±SD (stimulation index) |
|---|---|---|
| CMS001 | 10 | 1.7±0.2* |
| CMS003 | 10 | 1.6±0.2* |
| CMS007 | 8 | 1.7±0.1* |
| CMS034 | 9 | 1.5±0.2* |
| IFN-γ | 10 | 1.6±0.2* |
| IL-2 | 9 | 1.6±0.1* |
| Saline | 10 | 1.3±0.1 |
| *comparing to saline group P<0.05 | | |

At 0.5μg/kg/day, CMS008, CMS010, and CMS011 were found to be able to stimulate T lymphocyte transformation, having statistically significant difference from the saline group (P<0.05) as shown in Table 4.

Table 4

| Group | N | X±SD (stimulation index) |
|---|---|---|
| CMS008 | 10 | 1.7±0.3* |
| CMS010 | 9 | 1.7±0.3* |
| CMS011 | 10 | 1.6±0.4* |
| IFN-γ | 10 | 1.6±0.2* |
| IL-2 | 10 | 1.6±0.1* |
| Saline | 10 | 1.3±0:1 |
| * comparing to saline group P<0.05 | | |

2. The effect of peptide on NK cell cytotoxic activity

At 500μg/kg/day, CMS010, CMS013, CMS016, CMS023, CMS024, CMS026, CMS027, CMS028, CMS029, CMS030, CMS032, CMS033, CMS034, CMS035, and CMS036 were found to be able to increase NK cell cytotoxic activity, with statistically significant difference from the saline group (P<0.05). Among these peptides, CMS010, CMS016, and CMS030 were found to have statistically significant difference from the IFN-γ group and IL-2 group (P<0.05) as shown in Table 5.

Table 5

| Group | N | X±SD (%) |
|---|---|---|
| CMS010 | 9 | 91±4*@^ |
| CMS013 | 8 | 84±9* |
| CMS016 | 9 | 91±7*@^ |
| CMS023 | 10 | 79±12* |

(continued)

| Group | N | X±SD (%) |
|-------|---|----------|
| CMS024 | 10 | |
| CMS026 | 10 | 89±7* |
| CMS027 | 10 | 88±8* |
| CMS028 | 10 | 90±5* |
| CMS029 | 10 | 87±4* |
| CMS030 | 10 | 91±5*@^ |
| CMS032 | 10 | 87±5* |
| CMS033 | 9 | 89±8* |
| CMS034 | 11 | 85±9* |
| CMS035 | 8 | 90±10* |
| CMS036 | 10 | 88±7* |
| IFN-γ | 10 | 77±8* |
| IL-2 | 10 | 77±8* |
| Saline | 8 | 63 ±9 |

* comparing to saline group P<0.05
@ comparing to IFN-γ group P<0.05
^ comparing to IL-2 group P<0.05

At 50μg/kg/day, CMS001, CMS003, CMS015, CMS021, CMS026, and CMS035 were found to be able to increase NK cell cytotoxic activity, having statistically significant difference from the saline group (P<0.05). Among these peptides, CMS021 was found to have statistically significant difference from the IFN-γ group and IL-2 group (P<0.05). CMS012 was found to be able to inhibit NK cell cytotoxic activity, having statistically significant difference from the saline group (P<0.05). Data detailed in Table 6 below.

Table 6

| Group | N | X±SD (%) |
|-------|---|----------|
| CMS001 | 10 | 85±10* |
| CMS003 | 10 | 85±6* |
| CMS012 | 9 | 40±9* |
| CMS015 | 8 | 78±8* |
| CMS021 | 8 | 88±12*@^ |
| CMS026 | 10 | 76±9* |
| CMS035 | 10 | 72±9* |
| IFN-γ | 10 | 73±10* |
| IL-2 | 10 | 74±8* |
| Saline | 10 | 56±8 |

* comparing to saline group P<0.05
@ comparing to IFN-γ group P<0.05
^ comparing to IL-2 group P<0.05

At 5μg/kg/day, CMS008, CMS009, CMS010, CMS011, CMS012, CMS020, CMS024, CMS034, and CMS036 were found to be able to increase NK cell cytotoxic activity, having statistically significant difference from the saline group (P<0.05). Among these peptides, CMS008 and CMS009 were found to have statistically significant difference from the IFN-γ group and IL-2 group (P<0.05) as shown in Table 7.

Table 7

| Group | N | X±SD (%) |
|---|---|---|
| CMS008 | 10 | 92±4*@^ |
| CMS009 | 8 | 92±6*@^ |
| CMS010 | 10 | 82±9* |
| CMS011 | 10 | 76±10* |
| CMS012 | 10 | 85±7* |
| CMS020 | 9 | 91±6* |
| CMS024 | 9 | 78±3* |
| CMS034 | 8 | 90±5* |
| CMS036 | 10 | 75±9* |
| IFN-γ | 10 | 80±8* |
| IL-2 | 10 | 80±8* |
| Saline | 10 | 60±9 |

\* comparing to saline group P<0.05
@ comparing to IFN-γ group P<0.05
^ comparing to IL-2 group P<0.05

At 0.5μg/kg/day, CMS002, CMS011, CMS012, CMS022, CMS028, and CMS035 were found to be able to increase NK cell cytotoxic activity, having statistically significant difference from the saline group (P<0.05). CMS008 was found to be able to inhibit NK cell cytotoxic activity, having statistical significant difference from the saline group (P<0.05). Data detailed in Table 8 below.

Table 8

| Group | N | X±SD (%) |
|---|---|---|
| CMS002 | 8 | 76±9* |
| CMS008 | 10 | 46±12* |
| CMS011 | 9 | 79±3* |
| CMS012 | 9 | 77±6* |
| CMS022 | 10 | 73±11* |
| CMS028 | 8 | 79±3* |
| CMS035 | 10 | 76±10* |
| IFN-γ | 10 | 72±9* |
| IL-2 | 10 | 74±10* |
| Saline | 11 | 58±7 |

\* comparing to saline group P<0.05

3. The effect of peptides on the activity of T lymphocyte in secreting IL-2

At 500μg/kg/day, CMS007, CMS009, CMS010, and CMS015 were found to be able to promote IL-2 secretion from T lymphocyte, having statistically significant difference from the saline group (P<0.05). Among these peptides, CMS007 and CMS015 were found to have statistically significantly difference from the IL-2 group (P<0.05). And, CMS009 and CMS010 were also found to have statistically significant difference from both the IFN-γ group and IL-2 group (P<0.05) as shown in Table 9.

Table9

| Group | N | X±SD (IU) |
|---|---|---|
| CMS007 | 9 | 86±15*^ |
| CMS009 | 10 | 114±13*@^ |
| CMS010 | 9 | 125±17*@^ |
| CMS015 | 9 | 85±17*^ |
| IFN-γ | 10 | 100±18* |
| IL-2 | 10 | 70±13* |
| Saline | 10 | 39±10 |

\* comparing to saline group P<0.05
@ comparing to IFN-γ group P<0.05
^ comparing to IL-2 group P<0.05

At 50μg/kg/day, CMS001 and CMS003 were found to be able to promote activity of T lymphocyte in secreting IL-2, having statistically significant difference from the saline group (P<0.05). Among these peptides, CMS003 was found to have statistically significant difference from the.IL-2 group (P<0.05) as shown in Table 10.

Table 10

| Group | N | X±SD (IU) |
|---|---|---|
| CMS001 | 10 | 60±10* |
| CMS003 | 8 | 86±9*^ |
| IFN-γ | 9 - | 99±16* |
| IL-2 | 10 | 72±12* |
| Saline | 10 | 39±10 |

\* comparing to saline group P<0.05
^ comparing to IL-2 group P<0.05

At 5μg/day, CMS007, CMS012, and CMS020 were found to be able to promote T lymphocyte in secreting IL-2, having statistically significant difference from the saline group (P<0.05) as shown in Table 11.

Table 11

| Group | N | X±SD (IU) |
|---|---|---|
| CMS007 | 8 | 64±12* |
| CMS012 | 9 | 65±16* |
| CMS020 | 8 | 63±11 * |
| IFN-γ | 10 | 96±14* |
| IL-2 | 10 | 77±13* |
| Saline | 10 | 37±9 |

\* comparing to saline group P<0.05

At 0.5μg/kg/day, CMS010, CMS011, CMS012, CMS022; and CMS034 were found to be able to promote T lymphocyte in secreting IL-2, having statistically significant difference from the saline group (P<0.05). Among these peptides, CMS034 was found to have statistically significantly difference from the IL-2 group (P<0.05). And, CMS011 and CMS022 were also found to have statistically significant difference from both the IFN-γ group and IL-2 group (P<0.05) as shown in Table 12

Table 12

| Group | N | X±SD (IU) |
|---|---|---|
| CMS010 | 9 | 66±11* |
| CMS011 | 10 | 101±19*@^ |
| CMS012 | 8 | 59±13* |
| CMS022 | 9 | 109±14*@^ |
| CMS034 | 10 | 85±10*^ |
| IFN-γ | 10 | 87±15* |
| IL-2 | 10 | 73±13* |
| Saline | 10 | 38±13 |

\* comparing to saline group P<0.05

@ comparing to IFN-γ group P<0.05 ^ comparing to IL-2 group P<0.05

4. The effect of peptides on the activity ofT lymphocyte in secreting IFN

At 500 μg/kg/day, CMS010, CMS013, and CMS016 were found to be able to promote T lymphocyte in secreting interferon (IFN), having statistically significant difference from the saline group, IFN-γ group, and IL-2 group (P<0.05) as shown in Table 13.

Table 13

| Group | N | X±SD (IU) |
|---|---|---|
| CMS010 | 9 | 167±13*@^ |
| CMS013 | 9 | 154±15*@^ |
| CMS016 | 6 | 162±19*@^ |
| IFN-γ | 10 | 139±16* |
| IL-2 | 10 | 120±13* |
| Saline | 10 | 65±11 |

\* comparing to saline group P<0.05

@ comparing to IFN-γ group P<0.05 ^ comparing to IL-2 group P<0.05

At 50μg/kg/day, CMS001, CMS003, and CMS021 were found to be able to promote T lymphocyte in secreting IFN having statistically significant difference from the saline group (P<0.05). Among these peptides, CMS021 was found to have statistically significant difference from the IFN-γ group and IL-2 group (P<0.05) as shown in Table 14.

Table 14

| Group | N | X±SD (IU) |
|---|---|---|
| CMS001 | 10 | 110±15* |
| CMS003 | 8 . | 106±16* |
| CMS021 | 8 | 143±17*@^ |
| IFN-γ | 9 | 125±18* |
| IL-2 | 10 | 113±17* |

| (continued) | | |
|---|---|---|
| Group | N | X±SD (IU) |
| Saline | 10 | 61±11 |

* comparing to saline group P<0.05
@ comparing to IFN-γ group P<0.05
^ comparing to IL-2 group P<0.05

At 5μg/kg/day, CMS009 and CMS012 were found to be able to promote T lymphocyte in secreting IFN having statistically significant difference from the saline group (P<0.05). Among these peptides, CMS009 was found to have statistically significant difference from the IL-2 group (P<0.05) as shown in Table 15.

Table 15

| Group | N | X±SD (IU) |
|---|---|---|
| CMS009 | 10 | 121±15*^ |
| CMS012 | 9 | 86±9* |
| IL-2 | 9 | 105±14* |
| Saline | 10 | 66±10 |

* comparing to saline group P<0.05
^ comparing to IL-2 group P<0.05

At 0.5μg/kg/day, CMS010, CMS011, CMS022, and CMS028 were found to be able to promote T lymphocyte in secreting IFN, having statistically significant difference from the saline group (P<0.05). Among these peptides, CMS010 and CMS022 were found to have statistically significant difference from the IFN-γ group and IL-2 group (P<0.05) as shown in Table 16.

Table 16

| Group | N | X±SD (IU) |
|---|---|---|
| CMS010 | 9 | 142±18*@^ |
| CMS011 | 10 | 89±18* |
| CMS022 | 9 | 145±13*@^ |
| CMS028 | 10 | 96±13* |
| IFN-γ | 10 | 124±16* |
| IL-2 | 10 | 107±13* |
| Saline | 10 | 64±13 |

* comparing to saline group P<0.05
@ comparing to IFN-γ group P<0.05
^ comparing to IL-2 groupP<0.05

5. The effect of peptides on antibody formation

At 500 μg/kg/day, CMS002, CMS003, CMS007, CMS008, CMS009, CMS010, CMS011, CMS012, CMS013, CMS014, CMS015, CMS016, CMS018, CMS019, CMS020, CMS022, CMS023, CMS024, CMS029, MS033, and CMS035 were found to be able to promote anti-SRBC antibody formation, having statistically significant difference from the saline group (P<0.05). Among these peptides, CMS002, CMS003, CMS007, CMS008, CMS013, CMS019, CMS024, and CMS035 were found to have-statistically significant difference from the IFN-γ group (P<0.05). And, CMS009, CMS010, CMS011, CMS012, CMS014, CMS015, CMS016, CMS020, CMS023, CMS029, and CMS033 were also found to have statistically significant difference from both the IFN-γ group and IL-2 group (P<0.05) as shown in Table 17.

Table 17

| Group | N | X±SD (Unit) |
|---|---|---|
| CMS002 | 10 | 87±18*@ |
| CMS003 | 10 | 96±18*@ |
| CMS007 | 10 | 69±17*@ |
| CMS008 | 10 | 82±15*@ |
| CMS009 | 10 | 113±22*@^ |
| CMS010 | 10 | 112±30*@^ |
| CMS011 | 8 | 188±16*@^ |
| CMS012 | 8 | 141±21*@^ |
| CMS013 | 10 | 80±16*@ |
| CMS014 | 10 | 130±24*@^ |
| CMS015 | 10 | 136±24*@^ |
| CMS016 | 8 | 143±38*@^ |
| CMS018 | 10 | 66±16* |
| CMS019 | 10 | 91±26*@ |
| CMS020 | 6 | 155±35*@^ |
| CMS022 | 8 | 68±31* |
| CMS023 | 9 | 110±45*@^ |
| CMS024 | 8 | 75±29*@ |
| CMS029 | 8 | 115±22*@^ |
| CMS033 | 10 | 143±27*@^ |
| CMS035 | 10 | 88±16*@ |
| IFN-γ | 9 | 37±10 |
| IL-2 | 10 | 71±11* |
| Saline | 10 | 32±7 |

\* comparing to Saline group P<0.05

@ comparing to IFN-γ group P<0.05 ^ comparing to IL-2 group P<0.05

At 50μg/kg/day, CMS003, CMS011, CMS012, CMS013, CMS015, CMS021, CMS022, CMS023, CMS026, CMS027, CMS029, CMS030, CMS032, CMS033, CMS034, CMS035, and CMS036 were found to be able to promote anti-SRBC antibody formation, having statistically significant difference from the saline group (P<0.05). Among these peptides, CMS011, CMS013, and CMS015 were found to have statistically significant difference from the IFN-γ group (P<0.05). And, CMS021, CMS022, CMS023, CMS026, CMS027, CMS029, CMS030, CMS032, CMS033, CMS034, CMS035, and CMS036 were also found to have statistically significant difference from both the IFN-γ group and IL-2 group (P<0.05). CMS009 was found to be able to inhibit anti-SRBC antibody formation, having statistical significant difference from the saline group (P<0.05). Data detailed in Table 18 below.

Table 18

| Group | No | X±SD (Unit) |
|---|---|---|
| CMS003 | 10 | 52±11* |
| CMS009 | 8 | 13±5* |
| CMS011 | 9 | 67±9*@ |
| CMS012 | 8 | 50±14* |
| CMS013 | 8 | 70±9*@ |

(continued)

| Group | No | X±SD (Unit) |
|---|---|---|
| CMS015 | 10 | 54±9*@ |
| CMS021 | 9 | 94±20*@^ |
| CMS022 | 9 | 110±16*@^ |
| CMS023 | 8 | 84±11*@^ |
| CMS026 | 9 | 98±9*@^ |
| CMS027 | 9 | 93±11*@^ |
| CMS029 | 10 | 143±13*@^ |
| CMS030 | 10 | 141:33*@^ |
| CMS032 | 9 | 131±24*@^ |
| CMS033 | 8 | 112±15*@^ |
| CMS034 | 10 | 136±11*@^ |
| CMS035 | 8 | 97±10*@^ |
| CMS036 | 10 | 118±11*@^ |
| IFN-γ | 9 | 37±10 |
| IL-2 | 10 | 71±11* |
| Saline | 10 | 32±7 |

* comparing to saline group P<0.05

@ comparing to IFN-γ group P<0.05 ^ comparing to IL-2 group P<0.05

At 5μg/kg/day, CMS001, CMS003, CMS007, CMS008, CMS009, CMS011, CMS012, CMS013, CMS015, CMS016, CMS019, CMS020, CMS021, CMS023, CMS024, CMS026, CMS027, CMS028, CMS029, CMS030, CMS032, CMS033, CMS034, CMS035, and CMS036 were found to be able to promote anti-SRBC antibody formation, having statistically significant difference from the saline group (P<0.05). Among these peptides, CMS003, CMS008, CMS009, CMS012, CMS015, CMS016, CMS020, and CMS021 were found to have statistically significant difference from the IFN-γ group (P<0.05). And, CMS001, CMS007, CMS011, CMS019, CMS023, CMS024, CMS026, CMS027, CMS028, CMS029, CMS030, CMS032, CMS033, CMS034, CMS035, and CMS036 were also found to have statistically significant difference from both the IFN-γ group and IL-2 group (P<0.05) as shown in Table 19.

Table 19

| Group, | N | X±SD(Unit) |
|---|---|---|
| CMS001 | 9 | 110±24*@ |
| CMS003 | 9 | 91±24*@ |
| CMS007 | 9 | 122±12*@^ |
| CMS008 | 9 | 97±26*@ |
| CMS009 | 8 | 79±18*@ |
| CMS011 | 10 | 115±27*@^ |
| CMS012 | 10 | 81±22*@ |
| CMS013 | 10 | 93±28*@ |
| CMS015 | 8 | 94±37*@ |
| CMS016 | 9 | 93±32*@ |
| CMS019 | 10 | 118±20*@^ |
| CMS020 | 10 | 89±24*@ |
| CMS021 | 9 | 82±30*@ |
| CMS023 | 10 | 166±27*@^ |
| CMS024 | 7 | 171±39*@^ |
| CMS026 | 9 | 191±17*@^ |

(continued)

| Group, | N | X±SD(Unit) |
|---|---|---|
| CMS027 | 9 | 117±45*@^ |
| CMS028 | 10 | 121±48*@^ |
| CMS029 | 9 | 147±23*@^ |
| CMS030 | 9 | 158±37*@^ |
| CMS032 | 9 | 157±37*@^ |
| CMS033 | 7 | 128+39*@^ |
| CMS034 | 8 | 172±37*@^ |
| CMS035 | 9 | 176±39*@^ |
| CMS036 | 8 | 179±34*@^ |
| IFN-γ | 9 | 37±10 |
| IL-2 | 10 | 71±11* |
| Saline | 10 | 32±7 |

* comparing to saline group P<0.05
@ comparing to IFN-γ group P<0.05
^ comparing to IL-2 group P<0.05

At 0.5μg/kg/day, CMS021, CMS023, CMS024, CMS027, and CMS033 were found to be able to promote anti-SRBC antibody formation, having statistically significant difference from the saline group (P<0.05). Among these peptides, CMS021 and CMS033 were found to have statistically significant difference from the IFN-γ group (P<0.05). And, CMS023, CMS024, and CMS027 were also found to have statistically significant difference from both the IFN-γ group and IL-2 group (P<0.05). Also, CMS002, CMS003, CMS009, CMS010, CMS011, CMS013, CMS014, CMS015, CMS018, CMS019, CMS020, CMS026, CMS028, CMS029, CMS030, CMS034, and CMS036 were found to be able to inhibit anti-SRBC antibody formation, having statistically significant difference from the saline group (P<0.05). Data detailed in Table 20 below.

Table 20

| Group | N | X±SD (Unit) |
|---|---|---|
| CMS002 | 9 | 4±1* |
| CMS003 | 9 | 2±1* |
| CMS009 | 9 | 2±1* |
| CMS010 | 10 | 10±3* |
| CMS011 | 10 | 5±3* |
| CMS013 | 10 | 7±1* |
| CMS014 | 10 | 15±6* |
| CMS015 | 9 | 13±4* |
| CMS018 | 9 | 3±1* |
| CMS019 | 9 | 12±3* |
| CMS020 | 9 | 10±3* |
| CMS021 | 9 | 57±9*@ |
| CMS023 | 10 | 108±21*@^ |
| CMS024 | 10 | 98±6*@^ |
| CMS026 | 10 | 19±6* |
| CMS027 | 10 | 99±14*@^ |
| CMS028 | 10 | 18±5* |
| CMS029 | 9 | 18±7* |
| CMS030 | 9 | 19±7* |
| CMS033 | 9 | 78±12*@ |

| (continued) | | |
|---|---|---|
| Group | N | X±SD (Unit) |
| CMS034 | 10 | 20±2* |
| CMS036 | 9 | 20±6* |
| IFN-γ | 9 | 37±10 |
| IL-2 | 10 | 71±11* |
| Saline | 10 | 32±7 |

\* comparing to saline group P<0.05
@ comparing to IFN-γ group P<0.05
^ comparing to IL-2 group P<0.05

6. The effect of peptides on the phagocytotic activity of mononuclear phagocyte

At 500 μg/kg/day, CMS003, CMS008, CMS020, CMS022, and CMS024 were found to be able to enhance the phagocytotic activity of mononuclear phagocyte, having statistically significant difference from the saline group (P<0.05). Among these peptides, CMS022 was found to have statistically significant difference from the IFN-γ group and IL-2 group (P<0.05) as shown in Table 21.

| Table 21 | | |
|---|---|---|
| Group | N | X±SD (phagocytotic index) |
| CMS003 | 10 | 6.6±0.7* |
| CMS008 | 10 | 6.5±1.2* |
| CMS020 | 10 | 6.4±0.6* |
| CMS022 | 10 | 7.4±0.6*@^ |
| CMS024 | 10 | 6.4±1.0* |
| IFN-γ | 10 | 6.4±0.9* |
| IL-2 | 9 | 5.7±0.8 |
| Saline | 10 | 5.1±0.6 |

\* comparing to saline group P<0.05
@ comparing to IFN-γ group P<0.05
^ comparing to IL-2 group P<0.05

At 50μg/kg/day, CMS019, CMS024, and CMS030 were found to be able to enhance the phagocytotic activity of mononuclear phagocyte, having statistically significant difference from the saline group (P<0.05). Among these peptides, CMS019 was found to have statistically significant difference from the IL-2 group (P<0.05) as shown in Table 22.

| Table 22 | | |
|---|---|---|
| Group | N | X±SD (phagocytotic index) |
| CMS019 | 9 | 6.7±p.9*^ |
| CMS024 | 8 | 6.6±0.7* |
| CMS030 | 10 | 6.3±0.5* |
| IFN-γ | 10 | 6.4±0.9* |
| IL-2 | 9 | 5.7±0.8 |
| Saline | 10 | 5.1±0.6 |

\* comparing to saline group P<0.05
^ comparing to IL-2 group P<0.05

At 5μg/kg/day, CMS003*, CMS008, CMS009, CMS010, CMS011, CMS013, CMS016, CMS018, CMS019, and CMS035 were found to be able to enhance the phagocytotic activity of mononuclear phagocyte, having statistically significant difference from the saline group (P<0.05). Among these peptides, CMS003, CMS009, CMS010, CMS016, CMS019, and CMS035 were found to have statistically significant difference from the IL-2 group (P<0.05) as shown in Table 23.

Table 23

| Group | N | X±SD (phagocytotic index) |
|---|---|---|
| CMS003 | 9 | 6.9±0.9*^ |
| CMS008 | 9 | 6.4±0.5* |
| CMS009 | 9 | 6.9±0.9*^ |
| CMS010 | 10 | 7.1±0.7*^ |
| CMS011 | 10 | 6.4±1.1* |
| CMS013 | 10 | 6.7±0.2* |
| CMS016 | 9 | 6.9±0.8*^ |
| CMS018 | 8 | 6.7±1.2* |
| CMS019 | 8 | 6.8±0.6*^ |
| CMS035 | 9 | 6.9±0.9*^ |
| IFN-γ | 10 | 6.4±0.9* |
| IL-2 | 9 | 5.7±0.8 |
| Saline | 10 | 5.1 ±0.6 |

* comparing to saline group P<0.05
^ comparing to IL-2 group P<0.05

At 0.5μg/kg/day, CMS024, CMS027, and CMS036 were found to be able to enhance the phagocytotic activity of mononuclear phagocyte, having statistically significant difference from the saline group (P<0.05). Among these peptides, CMS024 was found to have statistically significant difference from the IL-2 group (P<0.05) as shown in Table 24.

Table 24

| Group | N | X±SD (phagocytotic index) |
|---|---|---|
| CMS024 | 10 | 6.7±0.5*^ |
| CMS027 | 10 | 6.4±0.6* |
| CMS036 | 9 | 6.2±0.3* |
| IFN-γ | 10 | 6.4±0.9* |
| IL-2 | 9 | 5.7±0.8 |
| Saline | 10 | 5.1±0.6 |

* comparing to saline group P<0.05
^ comparing to"IL-2 group P<0.05

7. The effect of peptides on the weight of immune organ

At 500μg/kg/day, CMS008, CMS010, CMS016, CMS019, CMS020, CMS022, CMS026, CMS027, CMS028, CMS029, CMS030, CMS032, CMS033, CMS034, CMS035, and CMS036 were found to be able to increase the weight of thymus gland, having statistically significant difference from the saline group (P<0.05). Among these peptides, CMS027 and CMS034. were found to have statistically significant difference from the IL-2 group (P<0.05). And, CMS008, CMS022, CMS029, CMS030, CMS032, CMS033, and CMS035 were also found to have statistically significant difference from both the IFN-γ group and IL-2 group (P<0.05) as shown in Table 25.

Table 25

| Group | N | X±SD (%) |
|---|---|---|
| CMS008 | 8 | 0.21±0.03*@^ |
| CMS010 | 10 | 0.19±0.04* |

(continued)

| Group | N | X±SD (%) |
|---|---|---|
| CMS016 | 9 | 0.18±0.05* |
| CMS019 | 10 | 0.19±0.02* |
| CMS020 | 10 | 0.19±0.04* |
| CMS022 | 9 | 0.26±0.05* |
| CMS026 | 10 | 0.20±0.03* |
| CMS027 | 8 | 0.20±0.03*^ |
| CMS028 | 10 | 0.19±0.02* |
| CMS029 | 10 | 0.22±0.04*@^ |
| CMS030 | 8 | 0.30±0.03*@^ |
| CMS032 | 8 | 0.25+0.03*@^ |
| CMS033 | 9 | 0.25±0.04*@^ |
| CMS034 | 9 | 0.20±0.05*^ |
| CMS035 | 10 | 0.21±0.03*@^ |
| CMS036 | 9 | 0.18±0.02* |
| IFN-γ | 10 | 0.15±0.04 |
| IL-2 | 9 | 0.14±0.03 |
| Saline | 9 | 0.12±0.02 |

* comparing to saline group P<0.05
@ comparing to IFN-γ group P<0.05
^ comparing to IL-2 group P<0.05

At 500μg/kg/day, CMS019 was found to be able to increase the weight of spleen, with statistically significant difference from the saline control groups (P<0.05) as shown in Table 26. CMS001, CMS003, CMS007, CMS009, CMS011, CMS013, CMS014, CMS015, CMS021, CMS023, CMS024, CMS027, and CMS036 were found to be able to decrease the weight of spleen, with statistically significant difference from the saline control group (P<0.05). Data detailed in Table 26 below.

Table 26

| Group | N | X±SD (%) |
|---|---|---|
| CMS001 | 10 | 0.43±0.07* |
| CMS003 | 8 | 0.40±0.04* |
| CMS007 | 9 " | 0.32±0.05* |
| CMS009 | 9 | 0.41±0.03* |
| CMS011 | 9 | 0.41±0.04* |
| CMS013 | 10 | 0.44±0.07* |
| CMS014 | 10 | 0.40±0.03* |
| CMS015 | 9 | 0.36±0.07* |
| CMS019 | 9 | 0.63±0.08* |
| CMS021 | 9 | 0.36±0.04* |
| CMS023 | 9 | 0.36±0.06* |
| CMS024 | 9 | 0.34±0.05* |
| CMS027 | 10 | 0.37±0.03* |
| CMS036 | 10 | 0.40±0.03* |
| Saline | 10 | 0.53±0.05 |

* comparing to saline group P<0.05

At 50 μg/kg/day, CMS002, CMS008, CMS012, CMS014, CMS016, CMS018, CM019, CMS020, CMS022, CMS023, CMS024, CMS026, CMS027, CMS028, CMS029, CMS030, CMS032, CMS033, CMS034, and CMS036 were found to be able to increase the weight of thymus gland, having statistically significant difference from the saline group (P<0.05). Among these peptides, CMS034 was found to have statistically significant difference from the IL-2 group (P<0.05). And, CMS002, CMS008, CMS012, CMS014, CMS016, CMS018, CMS019, CMS020, CMS022, CMS023, CMS024, CMS026, CMS027, CMS030, CMS032, and CMS036 were also found to have statistically significant difference from both the IFN-γ group and IL-2 group (P<0.05) as shown in Table 27.

Table 27

| Group | N | X±SD (%) |
|---|---|---|
| CMS002 | 10 | 0.21±0.02*@^ |
| CMS008 | 10 | 0.20±0.04*@^ |
| CMS012 | 10 | 0.26±0.02*@^ |
| CMS014 | 10 | 0.21 ±0.02*@^ |
| CMS016 | 10 | 0.20±0.03*@^ |
| CMS018 | 10 | 0.23±0.02*@^ |
| CMS019 | 10 | |
| CMS020 | 10 | 0.27±0.03*@^ |
| CMS022 | 10 | 0.30±0.03*@^ |
| CMS023 | 10 | 0.20±0.02*@^ |
| CMS024 | 10 | 0.27±0.02*@^ |
| CMS026 | 10 | 0.27±0.02*@^ |
| CMS027 | 8 | 0.21±0.03*@^ |
| CMS028 | 10 | 0.18±0.04* |
| CMS029 | 9 | 0.18±0.05* |
| CMS030 | 10 | 0.25±0.04*@^ |
| CMS032 | 10 | 0.27±0.03*@^ |
| CMS033 | 9 | 0.18±0.03* |
| CMS034 | 8 | 0.19±0.04*^ |
| CMS036 | 9 | 0.22±0.02*@^ |
| IFN-γ | 10 | 0.15±0.04 |
| IL-2 | 9 | 0.14±0.04 |
| Saline | 9 | 0.12±0.02 |

\* comparing to saline group P<0.05
@ comparing to IFN-γ group P<0.05
^ comparing to"IL-2 group P<0.05

At 50μg/kg/day, CMS008, CMS010, and CMS029 were found to be able to decrease the weight of spleen, with statistically significant difference from the saline control group (P<0.05). Data detailed in Table 28 below.

Table 28 .

| Group | N | X±SD (%) |
|---|---|---|
| CMS008 | 10 | 0.39±0.08* |
| CMS010 | 10 | 0.38±0.05* |
| CMS029 | 10 | 0.42±0.04* |
| IFN-γ | 10 | 0.50±0.04 |
| IL-2 | 9 | 0.62±0.07 |
| Saline | 9 | 0.53±0.05 |

\* comparing to saline group P<0.05

At 5μg/kg/day, CMS001, CMS002, CMS010, CMS011, CMS012, CMS013, CMS014, CMS015, CMS016, CMS018, CMS019, CMS020, CMS021, CMS022, CMS023, CMS024, CMS026, CMS028, CMS029, CMS030, CMS032, CMS033, CMS034, and CMS036 were found to be able to increase the weight of thymus gland, having statistically significant difference from the saline group (P<0.05). Among these peptides, CMS002, CMS014, CMS024, and CMS030 were found to have statistically significant difference from the IL-2 group (P<0.05). And, CMS010, CMS012, CMS018, CMS019, CMS020, CMS022, CMS026, CMS028, CMS032, CMS034, and CMS036 were also found to have statistically significant difference from both the IFN-γ group and IL-2 group (P<0.05) as shown in Table 29.

Table 29

| Group | N | X±SD (%) |
|---|---|---|
| CMS001 | 10 | 0.22±0.05* |
| CMS002 | 9 | 0.24±0.05*^ |
| CMS010 | 9 | 0.27±0.05*@^ |
| CMS011 | 10 | 0.22±0.04* |
| CMS012 | 10 | 0.27±0.06*@^ |
| CMS013 | 10 | 0.21±0.05* |
| CMS014 | 10 | 0.23±0.06*^ |
| CMS015 | 9 | 0.20±0.08* |
| CMS016 | 10 | 0.22±0.06* |
| CMS018 | 10 | 0.24±0.04*@^ |
| CMS019 | 10 | 0.24±0.02*@^ |
| CMS020 | 10 | 0.24±0.07*@^ |
| CMS021 | 9 | 0.20±0.06* |
| CMS022 | 9 | 0.25±0.04*@^ |
| CMS023 | 10 | 0.23±0.06* |
| CMS024 | 9 | 0.23±0.06*^ |
| CMS026 | 10 | 0.31±0.05*@^ |
| CMS028 | 10 | 0.28±0.06*@^ |
| CMS029 | 10 | 0.21±0.03* |
| CMS030 | 10 | 0.23±0.07*^ |
| CMS032 | 10 | 0.29±0.04*@^ |
| CMS033 | 10 | 0.20±0.02* |
| CMS034 | 9 | 0.27±0.06*@^ |
| CMS035 | 10 | 0.21±0.04* |
| CMS036 | 10 | 0.25±0.04*@^ |
| IFN-γ | 10 | 0.15±0.04 |
| IL-2 | 9 | 0.14±0.04 |
| Saline | 9 | 0.12±0.02 |

* comparing to saline group P<0.05
@ comparing to IFN-γ group P<0.05
^ comparing to IL-2 groupP<0.05

At 5μg/kg/day, CMS030 was found to be able to increase the weight of spleen, having statistically significant difference from the saline group (P<0.05). CMS015 was found to be able to decrease the weight of spleen, having statistically significant difference from the saline group (P<0.05). Data detailed in Table 30 below.

Table 30

| Group | N | X±SD(%) |
|---|---|---|
| CMS015 | 9 | 0.38±0.15* |
| CMS030 | 10 | 0.64±0.09* |

(continued)

| Group | N | X±SD(%) |
|-------|---|---------|
| Saline | 10 | 0.53±0.05 |

* comparing to saline group P<0.05

At 0.5μg/kg/day, CMS002, CMS008, CMS010, CMS012, CMS014, CMS018, CMS020, CMS022, CMS026, CMS028, CMS030, and CMS032 were found to be able to increase the weight of the thymus gland, having statistically significant difference from the saline group (P<0.05). Among these peptides, CMS008 and CMS012 were found to have statistically significant difference from the IL-2 group (P<0.05). And, CMS002, CMS020, and CMS030 were also found to have statistically significant difference from both the IFN-γ group and IL-2 group (P<0.05) as shown in Table 31.

Table 31

| Group | N | X±SD (%) |
|-------|---|----------|
| CMS002 | 8 | 0.26±0.06*@^ |
| CMS008 | 10 | 0.22±0.07*^ |
| CMS010 | 9 | 0.21±0.03* |
| CMS012 | 10 | 0.22±0.06*^ |
| CMS014 | 10 | 0.20±0.04* |
| CMS018 | 10 | 0.20±0.03* |
| CMS020 | 9 | 0.23±0.05*@^ |
| CMS022 | 10 | 0.21±0.06* |
| CMS026 | 9 | 0.21±0.05* |
| CMS028 | 10 | 0.20±0.06* |
| CMS030 | 8 | 0.24±0.05*@^ |
| CMS032 | 10 | 0.21±0.06* |
| IFN-γ | 10 | 0.15±0.04 |
| IL-2 | 9 | 0.14±0.04 |
| Saline | 9 | 0.12±0.02 |

* comparing to saline group P<0.05
@ comparing to IFN-γ group P<0.05
^ comparing to IL-2 groupP<0.05

At 0.5μg/kg/day, CMS020 was found to be able to increase the weight of spleen, having statistically significant difference from the saline group, IFN-γ group, and IL-2 group (P<0.05). CMS001 was found to be able to decrease the weight of spleen, having statistically significant difference from the saline group (P<0.05). Data detailed in Table 32 below.

Table 32

| Group | N | X±SD (%) |
|-------|---|----------|
| CMS001 | 10 | 0.40±0.05* |
| CMS020 | 8 | 0.68±0.09*@^ |
| Saline | 10 | 0.53±0.05 |
| IFN-γ | 10 | 0.50±0.04 |
| IL-2 | 10 | 0.62±0.07 |

* comparing to saline group P<0.05
@ comparing to IFN-γ group P<0.05
^ comparing to IL-2 group P<0.05

In summary, we found that peptides CMS001, CMS002, CMS003, CMS007, CMS008, CMS009, CMS010, CMS011, CMS012, CMS013, CMS014, CMS015, CMS016, CMS018, CMS019, CMS020, CMS021, CMS022, CMS023, CMS024, CMS026, CMS027, CMS028, CMS029, CMS030, CMS032, CMS033, CMS034, CMS035, and CMS036 have in vivo biological activities in the animal model tested.

## IV EFFECT OF CMS PEPTIDES ON CANCER

In order to find out whether these peptides have possible therapeutic on cancer, we used various standard animal cancer models in this study to test out the biological effects of the above peptides on diseased animals.

Materials

1. Experimental animals

BALB/c mice, $C_{57}BL/6$ mice, and DBA/2 mice, weighing 18-22g, from China Medical Science Institute, PR China.

2. Cell lines

Mouse sarcoma $S_{180}$ cells, $B_{16}$ cells, and **$L_{1210}$** cells, from Cancer Research Department, China Medical Science Institute.
YAC-1 cells, as gift from Prof Yao Zhi, Tianjin Medical University.

3. Main drug and reagent

The peptides used in this study were custom manufactured by American Peptide Company, Inc., USA.
Fetal bovine serum, RPMI-1640 cell culture medium, from Gibco, USA.
MTT, ConA, from Sigma, USA.
Recombinant mouse interferon-$\gamma$ (rmIFN-$\gamma$), from Beijing Biotech Inc., PR China.
Recombinant human interleukin-2 (rhIL-2), from Shanghai Huaxin Biotech Inc., PR China.
Lymphocyte separation solution, from Research Institute of Hematologic Disease, National Institute of Medical Science, PR China
Cyclophosphamide, from The 12the pharmaceutical factory of Shanghai, PR China.

Methods

1. Administration of test substance

Intraperitoneal injection, once per day. With the exception of cyclophosphamide group, all groups started treatment 5 days before transplanting the cancer cells. The cyclophosphamide groups were treated on the next day after transplanting the cancer cells. Treatment of all groups with testing substance lasted for 30 days or until the animal died unless otherwise specified.

2. The effect of peptides on the growth rate of transplanted **$S_{180}$** sarcoma cells in BALB/c mice, and that on the immunological function of the host

BALB/c mice were randomized into peptide group, cyclophosphamide group, rmIFN-$\gamma$ group, rhIL-2 group, and saline group, 20 animals per group.
Stock $S_{180}$ sarcoma cells were incubated in DMEM/F12 medium supplemented with 10% fetal bovine serum, 37°C, 5%$CO_2$ for 72 hrs, then washed 3-4 times with Hank's solution at room temperature. Adjust the cell concentration to $1-2 \times 10^9$ per litre with Hank's solution. 0.2ml of cell suspension was implanted to several BALB/c mice through the armpit for 10-12 days. The mice were killed by cervical vertebra dislocation. Vigorously growing and non-disrupted tumor masses were harvested and washed clean with sterile saline. The tissue was dispersed in saline to a homogenous cell suspension, in a ratio of 1g tissue to 4 ml saline. The sarcoma bearing mice model was prepared with an injection of 0.2ml cell suspension through the armpit [1]. Administration of test substance treatment started as described in section Method 1.
2.1 The effect of peptides on the phagocytotic function of mononuclear phagocyte in mice with $S_{180}$ sarcoma [2,3] was analysed by injecting mice from the tail vein with 0.1ml/10g body weight of Indian ink (1:5 dilution with normal saline) on the second day after the last test substance administration. At one minute and five minutes after the injection, 20$\mu$l blood was obtained from the eye canthus with heparinised tubing. The blood was mixed with 2ml 0.1% w/v $Na_2CO_3$ and then $OD_{680}$nm obtained. The outline clear index K was calculated by the following formula:

$$K = (\lg A_1 - \lg A_2) \div (t2 - t1)$$

Key:

Al: $OD_{680}$nm at first minute
A2: $OD_{680}$nm at fifth minute
t2: 5 minutes
t2: 1 minute

After the phagocytosis index study, the mice were killed by cervical vertebra dislocation. The liver, spleen, and cancer tissue were dissected, blotted dry, and weighed.

The phagocytosis index $\alpha$ was calculated as below:

$$\alpha = (^3\sqrt{K}) \times (W \div W_{LS})$$

key:

W: body weight
$W_{LS}$: weight of liver plus spleen

2.2 The tumor growth inhibition index was calculated according to the following formula:

Tumor growth inhibition index = (mean tumor weight of control group - mean tumor weight of treatment group) ÷ mean tumor weight of control group

3. The effect of peptides on the survival of BALB/c mice with transplanted ascitic fluid type liver cancer $H_{22}$

BALB/c mice were randomly grouped into peptide group, cyclophosphamide group, rmIFN-$\gamma$ group, rhIL-2 group and saline group, 20 animals per group.

Stock $H_{22}$ cells were incubated in DMEM/F12 medium supplemented with 10% fetal bovine serum, 37°C/5%$CO_2$ for 72 hrs, then washed 3-4 times with Hank's solution at room temperature. Adjust cell concentration to 1-2 x $10^9$ per litre with Hank's solution. 0.2ml of the cell suspension was implanted to the abdominal cavity of several BALB/c mice for 6-8 days [1]. The mice were killed by cervical vertebra dislocation. Ascitic fluid of the mice was collected aseptically and the cell concentration adjusted to 1 x $10^6$ per ml with Hank's solution. 0.2ml of the cell suspension was implanted into the abdominal cavity of healthy mice to generate the $H_{22}$ carrying mouse model for ascitic fluid type liver cancer. Administration of test substance started as described in section Method 1. Survival data of the mice was recorded. If the animal survived longer than the experiment, the survival days was recorded as the duration of the experiment. Mean survival day was obtained by the Kaplan-meier method in the Survival option of the SPSS software. The survival index was calculated according to the following formula:

$$\text{Survival index} = (\text{mean survival days in treatment group} - \text{mean survival days of control group}) \div \text{mean survival days of control group} \times 100\%$$

4. The effect of peptides on the cellular immunity of BALB/c mice with transplanted ascitic fluid type liver cancer $H_{22}$

4.1 Preparation of spleen cell suspension [1,4]

Healthy BALB/c mice were randomised into peptide group, rmIFN-$\gamma$ group, rhIL-2 group, and saline group, 15 mice per group, and prepared into $H_{22}$ carrying mice model as described in section Method 3. After implantation of the cancer cells, the test substances were administered for 15 days, the mice were killed by cervical dislocation. The spleen was isolated aseptically and manually dispersed in cold D-Hank's solution using an injection needle. The dispersed cell suspension was further sieved through a 100-gauge, 150$\mu$m diameter stainless steel sieve. After centrifugation at 200g for 10 minutes, the supernatant was discarded. The cell pellet was re-suspended in 10 volume of Tris-$NH_4$Cl buffer and

then kept standing still for 10 minutes at room temperature. The suspended cells were collected by centrifugation at 150g for 10 minutes. The cells were washed 2-4 times with cold D-Hank's solution by re-suspending and collecting by centrifugation with condition as described above. The washed cells were then diluted to the desired cell densities by RPMI-1640 culture medium, containing 10% fetal bovine serum.

4.2 The effect of peptides on the T lymphocyte transformation in mice with ascitic fluid type liver cancer $H_{22}$ [1,4]

Spleen cells of density $1 \times 10^6$/ml were placed onto a 96 wells cell culture plate, 100$\mu$l/well, three parallel wells each of the assay sample and control sample per mouse. To the assay wells, 100$\mu$l/well ConA of 100$\mu$g/ml in RPMI-1640 was added, and 100$\mu$l/well plain RPMI-1640 was used for the controls. The cells were incubated for 66 hrs at 37°C, 5% $CO_2$. The cells were then pelleted by centrifugation at 150g for 10 minutes. The supernatant was collected and stored at -20°C for cytokines IL-2 and IFN determination.

50$\mu$l/well MTT of 1mg/ml in RPMI-1640 was added to the cell pellet and the cells re-suspended by shaking for 2 minutes. The incubation was continued for 4 hours. The supernatant was discarded after centrifugation at 150g for 10 minutes. 120$\mu$l 40mM HCl-2-propanol was added to the cell pellet and shaken for 3 minutes. Use an ELISA reader to obtain $OD_{570}$nm of each well referenced at 630nm.

Each mouse formed three assay wells and three control wells. The Stimulation Index (SI) of each mouse was obtained by first deriving the average OD of the three parallel wells, then dividing the value of the assay wells by the control wells.

4.3 The effect of peptides on the NK cell activity in mice with ascitic fluid type liver cancer $H_{22}$ [5.6]

Mice spleen cells were prepared to $4x10^6$/ml as described in section 4.1 above. Target cells YAC-1 were brought to log phase and adjusted to $1x10^5$/ml. Using a 96 wells cell culture plate, 100$\mu$l mouse spleen cells and 100$\mu$l culture medium were added to the control well containing only the spleen cells; 100$\mu$l target cells and 100$\mu$l culture medium were added to the control well containing only target cells; 100$\mu$l mouse spleen cells and 100$\mu$l target cells were added to the NK activity assay well. Three parallel sets of the above were prepared per mouse. After that the 96 wells cell culture plates were incubated for 4hrs at 37°C, 5% $CO_2$.

Samples were centrifuge at 150g for 10 minutes to collect the cells. The supernatant was discarded and 50$\mu$l/well MTT of 1mg/ml was added. The reaction mixture was then shaken for 2 minutes, and incubated at 37°C, 5% $CO_2$ for 4 hours. The supernatant was discarded after centrifugation at 150g for 10 minutes. 120 $\mu$l 40mM HCl-2-propanol was added and shaken for 3 minutes. An ELISA reader was used to obtain $OD_{570nm}$ of each well referenced at 630nm.

Each mouse has 9 wells: three spleen cells only control, three target cells only control, and three assay wells with both spleen and target cells. The NK cell activity index of each mouse was obtained by first deriving the average OD of the three parallel wells of each combination, then applying this average OD to the following formula:

$$\text{NK cell activity index} = [1-(\text{average OD of spleen and target cell well} - \text{average OD of spleen cell only well}) \div (\text{average OD of target cell only well})] \times 100\%$$

5. The effect of peptides on the survival of DBA/2 mice with transplanted $L_{1210}$ leukemia

DBA/2 mice, 6-8 weeks old, were randomized into peptide group, cyclophosphamide group, rmIFN-$\gamma$ group, rhIL-2 group, and saline group, 20 animals per group.

Stock $L_{1210}$ cells were incubated in DMEM/F12 medium supplemented with 10% fetal bovine serum, 37°C/5%$CO_2$ for 72 hrs, then washed 3-4 times with Hank's solution, and adjusted to $1 \times 10^5$ cells per litre. 0.1ml of the cell suspension was implanted into the abdominal cavity of several healthy DBA mice for 6-8 days. The mice were then killed by cervical vertebra dislocation and their ascitic fluid collected aseptically. The cell concentration of the collected ascitic fluid was adjusted to 1 x 10 per ml with Hank's solution. 0.1ml of the cell suspension was inplanted into each of the testing animal and the survival data of the animals recorded. Treatment started in the testing animals as described in section Method 1. Mean survival day was obtained by the Kaplan-meier method in the Survival option of the SPSS software. If the animal survived longer than the experiment, the survival days was entered as the duration of the experiment. The survival index was calculated according to the following formula:

$$\text{Survival index} = (\text{mean survival days of treatment group} - \text{mean survival days of control group}) \div \text{mean survival days of control group}$$

6. The effect of peptides on the humoral immunity of $C_{57}BL/6$ mice bearing transplanted $B_{16}$ melanoma, and that on the metastatic potential of the inoculated melanoma cells

$C_{57}BL/6$ mice, 6-8 weeks old, body weight 18-22g, were randomized into peptide group, cyclophosphamide group, rmIFN-$\gamma$ group, rhIL-2 group, and saline group, 20 animals per group.

Stock $B_{16}$ mouse melanoma cells were incubated in DMEM/F12 medium supplemented with 10% fetal bovine serum, 37°C/5%$CO_2$ for 72 hrs, then washed 3-4 times with Hank's solution. The cell concentration was adjusted to 1 x $10^5$ per ml with Hank's solution and 0.1ml of the cell suspension was injected via the tail vein into the testing mice to generate the $B_{16}$ melanoma bearing animal model [7,8]. Treatment with test substance started as described in section Method 1.

6.1 The effect of peptides on the humoral immunity of $C_{57}BL/6$ mice bearing transplanted $B_{16}$ melanoma[9]

Sheep red blood cells (SRBC) were prepared by collecting blood from the cervical vein and put into a sterile flask with glass beads. The flask was shaken for 3 minutes and the blood then mixed with Alsever solution (glucose 2.05g, NaCl 0.4g, Na lemonade 0.8g, adjust to 100ml with distilled water) and stored at 4°C. Immediately before use, samples were centrifuged at 130g, 5 minutes to collect the SRBC. The cells were washed two times by re-suspension and centrifugation in normal saline. Then the cell pellet was collected by centrifugation at 180g for 10 minutes and re-suspended in saline to make the final working SRBC suspension, 2% (v/v).

Complement was prepared by adding 10 volumes of fresh Cavy serum into one volume centrifuge packed SRBC, and then gently shaked for 30 minutes at 4°C. The SRBC was removed by centrifugation at 200g for 10 minutes. 10 volumes of normal saline were added to obtain the working complement solution.

On the testing animals, on the 27th date of test substance treatment, 0.2ml of the working SRBC cell suspension was injected into each animal to raise antibody. On the day after the last test substance administration, blood was collected from the eye canthus and left at room temperature for one hour for serum exudation. After centrifugation at 200g for 10 minutes, the collected serum was diluted by 500 times with normal saline.

To 1ml diluted mouse serum of each mouse, 0.5ml SRBC suspension was added. Ice cold. Then 1ml working complement solution was added and incubated at 37°C water bath for 10 minutes. Reactions were terminated by ice cold. Samples were then centrifuged at 200g for 10 minutes to obtain the supernatant.

To 1ml of this supernatant, 3ml Drabkin solution was added and left at room temperature for 10 minutes. $OD_{540nm}$ was obtained.

Reference $OD_{540nm}$ was obtained by mixing 0.25ml SRBC suspension with Drabkin solution to 4ml and placed at room temperature for 10 minutes before $OD_{540nm}$ was taken.

$$\text{Hemolysis index} = (OD_{540nm} \text{ of test sample} \div \text{reference } OD_{540nm}) \times 500$$

6.2 After the humoral immunity study, the mice were killed by cervical vertebra dislocation. Postmortum examination of the animals was performed. The pathological changes were recorded, and the numbers of melanoma lung metastasis foci were counted.

Results

1. The effect of peptide on the cellular phagocytosis in BALB/c mice with transplanted $S_{180}$ sarcoma (Method 2.1)

Table IV.1. The effect of peptide on the phagocytosis index of BALB/c mice with transplanted $S_{180}$ sarcoma

| Group | Dose | N | Phagocytosis index |
|-------|------|---|--------------------|
| CMS001 | 50$\mu$g/kg | 20 | 6.24$\pm$0.33*^ |

(continued)

| Group | Dose | N | Phagocytosis index |
|---|---|---|---|
| CMS001 | 5µg/kg | 19 | 6.67±0.43*^ |
| CMS034 | 5µg/kg | 19 | 6.20±0.44*^ |
| CMS034 | 0.5µg/kg | 20 | 6.35±1.02* |
| IL-2 | $3\times10^5$IU/kg | 19 | 6.96±1.37* |
| IFN-γ | 3x $10^5$IU/kg | 17 | 5.45±0.71 |
| Cyclo-phos phamide | 20mg/kg | 19 | 5.92±2.47 |
| Saline | 0.5ml | 19 | 5.38±0.85 |

* : comparing to saline, P<0.05
^ : comparing to rmIFN-γ, P<0.05

CMS001 at 50µg/kg/day and 5µg/kg/day, and CMS034 at 5µg/kg/day and 0.5µg/kg/day were found to be able to increase the phagocytosis index, having statistically significant difference from the saline group.

2. The effect of peptide on the growth rate of transplanted $S_{180}$ sarcoma in BALB/c mice (Method 2.2)

Table IV.2. The effect of peptide on transplanted S 180 tumor growth

| Group | Dose | N | Tumor weight(g) | Tumor inhibition index (%) |
|---|---|---|---|---|
| CMS010 | 500µg/kg | 20 | 0.67±0.35* | 48.4 |
| CMS034 | 0.5µg/kg | 20 | 0.83±0.48* | 35.9 |
| CMS035 | 5µg/kg | 20 | 0.71±0.37* | 44.6 |
| IL-2 | $3\times10^5$IU/kg | 20 | 0.69±0.37* | 46.2 |
| IFN-γ | $3\times10^5$IU/kg | 18 | 0.96±0.45 | 25.3 |
| cyclo-phos phamide | 20mg/kg | 20 | 0.68±0.32* | 47.3 |
| Saline | 0.5ml | 20 | 1.29±0.50 | |

* comparing to saline group, P<0.05

CMS010 at 500µg/kg/day, CMS034 at 0.5µg/day, and CMS035 at 5µg/kg/day were found to be able to reduce the growth of the transplanted $S_{180}$ sarcoma, having statistically significant difference from the saline group (P < 0.05).

3. The effect of peptide on the survival of BALB/c mice with transplanted ascitic fluid type liver caner $H_{22}$ (Method 3)

Table IV.3. The effect of peptide on the survival index of BALB/c mice with transplanted ascitic fluid type liver cancer H22

| Group | Dose | N | Survival days | Survival index (%) |
|---|---|---|---|---|
| CMS008 | 5µg/kg | 20 | 50.7±20.9*& | 67.8 |
| CMS011 | 5µg/kg | 20 | 36.4±22.2*& | 60.2 |
| CMS024 | 50µg/kg | 20 | 36.3±12.7*&$ | 38.4 |
| CMS024 | 5µg/kg | 19 | 40.6±14.6*&$ | 54.8 |
| CMS024 | 0.5µg/kg | 19 | 46.4±14.8*^&$ | 76.9 |
| CMS032 | 0.5µg/kg | 20 | 42.8±12.2*^&$ | 63.3 |
| rhIL-2 | $3\times10^5$IU/kg | 18 | 13.6±0.5 | |
| rmIFN-γ | $3\times10^5$IU/kg | 20 | 27.8±7.5 | 6.1 |
| Cyclo-phos phamide | 20mg/kg | 20 | 24.7±10.2 | |

(continued)

| Group | Dose | N | Survival days | Survival index (%) |
|---|---|---|---|---|
| Saline | 0.5ml | 19 | 26.2±6.8 | |

\* : comparing to saline, P<0.05
^: comparing to rmIFN-γ, P<0.05
& : comparing to rhIL-2, P<0.05
$ : comparing to cyclophosphamide, P<0.05

CMS008 at 5μg/kg/day, CMS011 at 5μg/kg/day, CMS024 at 50μg/kg/day, CMS024 at 0.5μg/kg/day, and CMS032 at 0.5μg/kg/day were found to be able to prolong the survival of BALB/c mice with transplanted $H_{22}$ ascitic fluid type liver cancer, having statistically significant difference from the saline group (P<0.05). It was also observed that in the group CMS024 0.5μg/kg/day, more than 30% (n=6) of the mice could survive longer than 90 days (two months after the experiment ended). Postmortem examination of the mice did not show sign of tumor establishment. CMS024 at 0.5μg/kg/day therefore may interfere with the growth of the transplanted $H_{22}$ by interfering with its establishment or inducing the complete healing of the established cancer.

4. The effect of peptide on T lymphocyte transformation in BALB/c mice with transplanted ascitic fluid type liver cancer $H_{22}$ (Method 4.2)

Table IV.4. The effect of peptide on T lymphocyte transformation

| Group | Dose | N | Stimulation index |
|---|---|---|---|
| CMS010 | 500μg/kg | 20 | 1.45±0.21*$ |
| CMS019 | 0.5μg/kg | 19 | 1.50±0.19*$ |
| CMS024 | 0.5μg/kg | 19 | 1.46±0.19*$ |
| CMS024 | 5μg/kg | 20 | 1.45±0.21*$ |
| CMS034 | 0.5μg/kg | 20 | 1.37±0.10*$ |
| CMS035 | 0.5μg/kg | 20 | 1.40±0.13*$ |
| CMS035 | 5μg/kg | 20 | 1.46±0.16*$ |
| rhIL-2 | $3×10^5$IU/kg | 19 | 1.46±0.21* |
| rmIFN-γ | $3×10^5$IU/kg | 18 | 1.27±0.14 |
| Cyclo-phosphamide | 20mg/kg | 19 | 1.01±0.23* |
| Saline | 0.5ml | 20 | 1.25±0.07 |

\*: comparing to saline, P<0.05
$ : comparing to cyclophosphamide, P<0.05

CMS010 at 500μg/kg/day, CMS019 at 0.5μg/kg/day, CMS024 at 0.5μg/kg/day and 5μg/kg/day, and CMS035 at 0.5μg/kg/day and 5μg/kg/day were found to be able to increase the stimulation index of T-lymphocyte, having statistically significant difference from the saline group (P<0.05).

5. The effect of peptide on the NK cell activity in BALB/c mice with transplanted ascitic fluid type liver cancer $H_{22}$ (Method 4.3)

Table IV.5. The effect of peptide on the NK cell cytotoxic activity index

| Group | Dose | N | NK activity index(%) |
|---|---|---|---|
| CMS003 | 500μg/kg | 17 | 37.9±14.5*^$ |
| CMS014 | 0.5μg/kg | 17 | 40.7±19.7*$ |
| CMS024 | 0.5μg/kg | 18 | 39.3±18.7*$ |
| CMS024 | 5μg/kg | 20 | 34.9±12.1*^$ |

(continued)

| Group | Dose | N | NK activity index(%) |
|---|---|---|---|
| CMS024 | 50μg/kg | 20 | 43.6±13.9*^$& |
| CMS032 | 5μg/kg | 20 | 52.6±12.5*^$& |
| CMS032 | 50μg/kg | 19 | 41.0±18.7*^$& |
| CMS034 | 50μg/kg | 20 | 57.3±17.9*^$& |
| IL-2 | 3×10⁵IU/kg | 19 | 26.0±9.0 |
| IFN-γ | 3×10⁵IU/kg | 18 | 20.9±3.3 |
| Cyclo-phos Phamide | 20mg/kg | 19 | 16.5±7.2* |
| Saline | 0.5ml | 20 | 24.0±8.2 |

* : as compared with saline, P<0.05.
^ : as compared with IFN-γ, P<0.05.
& : as compared with IL-2, P<0.05.
$ : as compared with cyclophosphamide, P<0.05

CMS003 at 500μg/kg/day, CMS014 at 0.5μg/kg/day, CMS024 at 0.5μg/kg/day, 5μg/kg/day, and 50μg/kg/day, and CMS034 at 50μg/kg/day were found to be able to increase the NK cell cytotoxic activity in the testing animal model, having statistically significant difference from the saline group (P<0.05).

6. The effect of peptide on the survival of DBA/2 mice with transplanted $L_{1210}$ leukemia (Method 5)

Table IV.6. The effect of peptide on the survival index of testing animals

| Group | Dose | N | Survival days | Survival index (%) |
|---|---|---|---|---|
| CMS019 | 0.5μg/kg | 20 | 21.1±5.8* | 26.8 |
| CMS035 | 0.5μg/kg | 20 | 29.3±15.4* | 76.1 |
| IL-2 | 3×10⁵IU/kg | 20 | 32.0±13.7* | 92.3 |
| IFN-γ | 3×10⁵IU/kg | 20 | 15.6±2.2 | |
| Cyclophos phamide | 20mg/kg | 20 | 24.0±5.3* | 44.2 |
| Saline | 0.5ml | 21 | 16.6±5.6 | |

* : comparing to saline group, P<0.05

CMS019 at 0.5μg/kg/day and CMS035 at 0.5μg/kg/day were found to be able to prolong the survival of DBA/2 mice with transplanted L1210 leukemia, having statistically significant difference from the saline group (P<0.05).

7. The effect of peptide on the humoral immunity of $C_{57}BL/6$ mice with transplanted $B_{16}$ melanoma (Method 6.1)

Table IV.7. The effect of peptide on the hemolysis index of C57BL/6 mice with transplanted B16 melanoma

| Group | Dose | N | Hemolysis index |
|---|---|---|---|
| CMS001 | 0.5μg/kg | 20 | 51.0±16.2*$ |
| CMS001 | 5μg/kg | 20 | 41.3±17.7*$ |
| CMS001 | 50μg/kg | 19 | 45.0±31.9*$ |
| CMS001 | 500μg/kg | 20 | 36.0±10.2*$ |
| CMS003 | 0.5μg/kg | 20 | 61.6±26.9*$ |
| CMS003 | 5μg/kg | 20 | 37.2±15.9*$ |
| CMS003 | 50μg/kg | 20 | 38.7±13.5*$ |
| CMS003 | 500μg/kg | 20 | 35.9±13.0*$ |
| CMS008 | 0.5μg/kg | 19 | 42.7±18.4*$ |

(continued)

| Group | Dose | N | Hemolysis index |
|---|---|---|---|
| CMS008 | 5μg/kg | 20 | 38.9±12.0*$ |
| CMS008 | 50μg/kg | 20 | 37.1±16.7*$ |
| CMS008 | 500μg/kg | 20 | 50.1±17.8*$ |
| CMS010 | 0.5μg/kg | 18 | 34.9±10.5*$ |
| CMS010 | 5μg/kg | 20 | 51.0±14.6*$ |
| CMS010 | 50μg/kg | 20 | 39.6±7.7*$ |
| CMS010 | 500μg/kg | 20 | 50.1±16.7*$ |
| CMS011 | 0.5μg/kg | 20 | 32.0±14.7* |
| CMS011 | 500μg/kg | 20 | 34.4±19.4* |
| CMS016 | 500μg/kg | 20 | 43.3±29.9* |
| CMS019 | 0.5μg/kg | 20 | 42.0±12.0*$ |
| CMS019 | 5μg/kg | 20 | 35.4±15.1*$ |
| CMS019 | 50μg/kg | 20 | 28.3±7.6* |
| CMS024 | 0.5μg/kg | 20 | 43.0±10.7*$ |
| CMS024 | 5μg/kg | 20 | 42.2±11.8*$ |
| CMS024 | 50μg/kg | 20 | 27.8±9.1* |
| CMS024 | 500μg/kg | 18 | 30.1±10.0* |
| CMS034 | 0.5μg/kg | 18 | 50.8±18.4*$ |
| CMS034 | 5μg/kg | 19 | 43.0±11.7*$ |
| CMS034 | 50μg/kg | 20 | 30.2±10.9* |
| CMS035 | 5μg/kg | 20 | 38.9±21.2*$ |
| CMS035 | 50μg/kg | 20 | 44.7±22.7*$ |
| CMS035 | 500μg/kg | 19 | 40.5±25.8* |
| rhIL-2 | $3\times10^5$IU/kg | 19 | 49.3±24.7* |
| rmIFN-γ | $3\times10^5$IU/kg | 19 | 60.5±17.4* |
| Cyclo-phos phamide | 20mg/kg | 19 | 20.7±19.1 |
| Saline | 0.5ml | 20 | 19.0±9.1 |

\* : comparing to saline, P<0.05

^: comparing to rmIFN-γ, P<0.05

& : comparing to rhIL-2, P<0.05

$ : comparing to cyclophosphamide, P<0.05

CMS001, CMS003, CMS008, CMS010, CMS011, CMS016, CMS019, CMS024, CMS034, and CMS035 were found to be able to enhance the humoral response (increase in hemolysis index) of $C_{57}$BL/6 mice with transplanted $B_{16}$ melanoma at dosages as shown on Table IV.7, having statistically significant difference from the saline group (P<0.05).

8. The effect of peptide on the survival of inoculated B16 melamona cells in $C_{57}$BL/6 mice (Method 6.2)

Postmortum examination of animals after the ending of test substance treatment did not show any sign of existance of B16 metastatic foci in the lung of mice treated with CMS008 at 0.5μg/kg/day, 5μg/kg/day, and 50μg/kg/day, and CMS016 at 5μg/kg/day and 500μg/kg/day.

Conclusion

In compliance with the Preclinical New Drug Research Guidelines issued by Branch of Drug Administration, Department of Health, PR China in 1993, the effects of peptide on mice with transplanted cancer cells was studied. It was concluded that

1. CMS010, CMS034, and CMS035 at suitable dosage could significantly inhibit the development of transplanted $S_{180}$ sarcoma in mice;

2. CMS001 and CMS034 at suitable dosage could enhance the phagocytotic immune activities of mice transplanted with $S_{180}$ sarcoma;

3. CMS008, CMS011, CMS024, and CMS032 at suitable dosage could prolong the survival of mice transplanted with ascitic fluid type liver cancer H22;

4. CMS010, CMS019, CMS024, CMS034, and CMS035 at suitable dosage could enhance T lymphocyte transformation in mice transplanted with ascitic fluid type liver cancer H22;

5. CMS003, CMS014, CMS024, CMS032, and CMS034 at suitable dosage could increase the NK cell cytotoxic activity of mice transplanted with ascitic fluid type liver cancer H22;

6. CMS019 and CMS035 at suitable dosage could prolong the survival of mice transplanted with L1210 leukemia;

7. CMS008 and CMS016 at suitable dosage could inhibit the development of transplanted B16 melanoma in mice;

8. CMS001, CMS003, CMS008, CMS010, CMS011, CMS016, CMS019, CMS024, CMS034, and CMS035 at suitable dosage could enhance the humoral immune response of mice transplanted with B16 melanoma.

Discussion

CMS001, CMS003, CMS008, CMS010, CMS011, CMS014, CMS016, CMS019, CMS024, CMS032, CMS034, CMS035 may be useful as a part, or on its own, in cancer management in human. For example, CMS001, CMS003, CMS008, CMS010, CMS011, CMS014, CMS016, CMS019, CMS024, CMS032, CMS034, and CMS035 may be used to increase the immunity of cancer patients. CMS008, CMS010, CMS016, CMS034, and CMS035 may be used to interfer with the growth of cancer cells in patients. CMS008, CMS011, CMS019, CMS024, CMS032, and CMS035 may be used to prolong the life expectancy of cancer patients. The peptides may be used on its own, in combinations of two or more peptides, or in combination with other pharmaceuticals or food supplements as a total course for cancer management.

Table IV. 8 Peptides Effective for Cancer

| CMS code | SEQ ID No. |
| --- | --- |
| CMS001 | 1 |
| CMS003 | 27 |
| CMS008 | 3 |
| CMS010 | 4 |
| CMS011 | 30 |
| CMS014 | 7 |
| CMS016 | 9 |
| CMS019 | 11 |
| CMS024 | 16 |
| CMS032 | 22 |
| CMS034 | 24 |
| CMS035 | 25 |

Reference

1. Principles of Pre-clinical Research of New Drugs, People's Republic of China. 1993, 7:137-143

2. Principles of Pre-clinical Research of New Drugs, People's Republic of China. 1993, 7:128-129

3. Yuanpei Zhang, Huaide Su. Pharmacological experiment (second edition). People's Health Publishing House. 1998, 137-138

4. Shuyun Xu, Rulian Bian, Xiu Chen. Methodology of pharmacological experiment. People's Health Publishing House. 1991, 1221-1234

5. Principles of Pre-clinical Research of New Drugs, People's Republic of China. 1993, 7:140

6. Jinsheng He, Ruizhu Li, Tingyi Zong. The study on MTT reduction method of testing NK cell activity. China Immunology Journal. 1996, 1(6): 356-358

7. Yaoqin Yang, Huchuan Yang, Huihong Tao, et al. The synergic effect of Tween-80 on the antitumor of hyperthermia-Experimental studies of mouse melanoma. Cancer Research on Prevention and Treatment. 1999,26(4): 8-12

8. Jian Fu, Jie Zheng, Weigang Fang, et al. Interleukin-12 gene transfection into murine B16 melanoma cells suppresses tumorigenicity and decreases metastatic potential. National Medical Journal of China. 1998,78(8): 627-629

9. Qian Wang. Modem medical experiment method. People's Health Publishing House. 1998, 482-483

10. Qichao Pan, Bin Xu. Cancer pharmacology and chemotherapy. Henan medical university Publishing House. 2000,66-69

11. Yuanpei Zhang, Huaide Su. Pharmacological experiment (second edition). People's Health Publishing House. 1998,131

The use of the above-identified peptide, in pharmaceutical formulations may be employed as possible treatment for immunological disorders or disease having secondary effect on immunity e.g. cancer The formulations may contain the peptide mixed with other active or inactive constituents, including other peptides. E.g. two to several (e.g. 3-5) of the listed peptides may be added to the same formulation with or without other ingredients. Alternatively, the peptide may be used to prepare the formulation together with peptides not listed here. They can be administered in the form of intravenous, intramuscular, intracutaneous, subcutaneous or intradermal. The mode of administration may also be intra-arterial injection that leads directly to the organ of problem.. Other modes of administration are transdermal, inhalation as powder or spray, and other forms of delivery known by one in the art. The formulation may also be orally taken, and may contain carriers that can be used to prevent_gastric digestion of the peptide after oral intake or any other carriers known in the art (for transdermal such as liposome).

The pharmaceutical formulation may include any of the knows pharmaceutical carriers. Examples of suitable carriers include any of the standard pharmaceutically accepted carrier known to those skilled in the art. These include but are not limited to, physiological saline solution, water, emulsions including oil and water mixtures or triglyceride emulsions, and other types of agents, fillers, coated tablets and capsules. The appropriate carrier may be selected based on the mode of administration of the pharmaceutical composition.

The peptide may be administered via intravenous injection, intramuscular injection, intraperitoneal injection, subcutaneous injection, and subcutaneous implantation. The peptide may also be administered in any form of oral administration like tablet, capsule, suspension, solution etc, in the usual form without modification or in slow release form, or with or without gastro-enteric protection. The peptide may further be applied in any form of topic application like ointment, cream, gel etc with or without transdermal facilitating device. The peptide may also be interpreted into its genetic sequence and cloned into an expression system, on its own or in combination with other peptide sequences, to generate a resulting peptide molecule to make use of the activity of the peptide as described in this report.

The dose of each peptide may be 1ng - 10g per kg body weight. A preferred dose is 10ng - 10mg per kg, and more preferably 1$\mu$g - 1mg per kg for an injection mode of administration. However, the effective dose can be as low as 1ng per kg body weight, since one or more of the peptides may operate through receptors that will induce a cascade of normal physiological response. Alternatively, one or more of the peptides can just be an initiator for a whole cascade of reaction. For an oral intake, the amount may be 1ng - 10g per day per kg body weight, more preferably 0.1$\mu$g - 1g per day per kg body weight and even more preferably 1$\mu$g - 10mg per day.

III Gene Therapy and Method of Treatment

Gene therapy based on the discovered peptide sequences is performed by designing a nucleic acid sequence that code for the peptide. The nucleic acid may be synthesized chemically and operably ligated to a promoter, and cloned into an expression vector. The expression vector is then administered into the human body as the form of gene therapy for expression in the human cell. The term "genetic vectors" as used herein includes these expression vectors. Vectors that can be used for gene therapy includes adeno-associated virus (Mizuno, M. et al. (1998). Jpn J Cancer Res 89, 76-80), LNSX vectors (Miller, A.D. et al. (1993) Methods Enzymol 217, 581-599) and lentivirus (Goldman, M.J. et al. (1997) Hum Gene Ther 8,2261-2268).

Other vehicles for peptide delivery include expression vectors encoding the desired peptide that can be transferred into an organism which can replicate in the host organism to which it is desired to administer the peptide without significant detrimental effects on the health of the host organism. For example, the expression vectors may be transferred into an organism which is not pathogenic to the host organism to which it is desired to administer the peptide. The expression vector produces the desired peptide in a bacterial or fungal organism which does not have significant detrimental effects on the health of the host organism to which the peptide is to be administered. For example, the expression vector encoding the desired peptide may be an expression vector which produces the desired peptide in an organism such as lactic acid bacteria, E. coli, or yeast. The expression vector produces the desired peptide in a microbe normally found in the mammalian gut or a microbe tolerated by the mammalian digestive tract. Some of the microbial species in which the desired peptide can be expressed include, but are not limited to, *Lactobacillus* species, such as *L. acidophilus, L. amylovorus, L. casei, L. crispatus, L. gallinarum, L. gasseri, L. johnsonii, L. paracasei, L. plantarum, L. reuteri, L. rhamnosus* or others; *Bifidobacterium* species, such as *B. adolescentis, B. animalus, B. bifidum, B. breve, B. infantis, B. lactis, B. longum* or others; *Enterococcus faecalis* or *Ent. facium ; Sporolactobacillus inulinus ; Bacillus subtilis* or *Bacillus cereus ; Escherichia coli ; Propionibacterium freudenreichii ;* or *saccharomyces cerevisiae* or *Saccharomyces-boulardii.*

Nucleic acid sequences that encode the peptide used in the present invention, chemically synthesized or produced by other means, including but not limited to the reverse transcription of mRNA to produce cDNA molecules, are incorporated into expression vectors for gene transfer into the desired organisms by methods of genetic engineering familiar to those of skill in the art. The expression vectors may be DNA vectors or RNA vectors. For example, the expression vectors may be based on plasmid or viral genetic elements. The expression vectors may be vectors which replicate extra-chromosomally or vectors which integrate into the chromosome.

The expression vectors comprise a promoter operably linked to a nucleic acid encoding a peptide used in the present invention. The promoter may be a regulatable promoter, such as an inducible promoter, or a constitutive promoter. The promoter may be selected to provide a desired level of peptide expression. In addition, if desired, the expression vectors may comprise other sequences to promote the production, presentation and/or secretion of peptides. For example, a nucleic acid encoding a peptide used in the present invention is operably linked to a nucleic acid sequence which directs the secretion of the peptide, exemplified by, but not limited to a nucleic acid encoding a signal peptide.

The expression vectors which are engineered to encode the peptide used in the present invention may be expression vectors which are adapted for expressing the peptide used in the present invention in a bacterial species that makes up the normal gut flora of mammals, such as *Lactobacillus* species and *Bacillus subtilis*. Examples of such expression vectors can be found in US Patents No. 6,100, 388, to Casas, and No. 5, 728, 571, to Bellini, respectively. It will be appreciated that any expression vector which facilitates the expression of a peptide used in the present invention in an organism which is not detrimental to the health of the host organism to which the peptide is to be administered may be used.

The expression vectors which are engineered to encode the peptide used in the present invention may be expression vectors which are adapted for expressing the peptide in a yeast species that is well tolerated by the mammaliam gut, such as *Saccharomyces cerevisiae* ; or, preferably, *Saccharomyces boulardii*, which can colonize the human gut and is used to treat certain forms of diarrhea. Yeast expression vectors can be used that constitutively express heterologous proteins and peptides, are highly stable, thus are well transmitted to progeny cells during mitosis and meiosis and may comprise coding sequence for a signal peptide or peptides that direct high levels of recombinant protein secretion. An example of such a yeast vector is given in US Patent No. 6,391, 585, to Jang et al.

The expression vectors encoding the peptide useful in the present invention may be introduced into the organism in which it is intended to express the peptides through techniques known in the art. These techniques include traditional methods of transforming bacteria, yeast, or other microbes, through the use of chemically competent bacterial cells electroporation or lithium acetate transformation (for yeast), for example, as well as recent advances in the transformation of bacterial species recalcitrant to these procedures. The expression vectors are introduced into lactic acid bacteria known to be recalcitrant to transformation using the method disclosed by Leer et al. (WO 95/35389) . The introduced sequences may be incorporated into microbial chromosomal DNA or may remain as extrachromosomal DNA elements.

This genetically engineered microbe containing the expression vector can then be inoculated into the alimentary canal, vagina, trachea etc. to achieve sustained immuno-therapy. The organisms expressing the peptides are ingested in an inactive form or, preferably, in live form. In the gut these microorganisms produce said peptides, release them into the lumen by secretion or by lysis of the microorganism or otherwise present the peptides to the host, whereby the peptides produce their intended effect upon the host organism. Peptides are presented to the host at the mucous membrane of the nasal passages, vagina or the small intestine.

another method of the treatment is the use of liposomes as a means for delivering the specific nucleic acid to the cells in the human body. The nucleic acid (such as an expression vector containing a nucleic sequence that encodes peptides of sequence ID No. 16) is delivered in an environment that encourages cellular uptake and chromosomal incorporation as described in Gao, X. and Huang, L. (1995) Gene Ther 2, 710-722 and US 6,207,456. Alternatively, the peptide itself can be encapsulated in the liposome and delivered directly, using a method described in US6,245,427.

The nucleic acid sequences useful for the above-mentioned gene therapy and method of treatment include sequences that code for these peptides Any one of the numerous nucleic acid sequences may be used to code for these peptides based on the degenerate codon system.

References

1. Principles of Pre-clinical Research of New Drugs, People's Republic of China. 1993, 7:134-135

2. Shuyun Xu, Rulian Bian, Xiu Chen. Methodology of pharmacological experiment. People's Health Publishing House. 1991, 1221-1234

3. Principle of new drug research in pre-clinic issued by Ministry of Health, People's Republic of China. 1993, 7:140

4. Jinsheng He, Ruizhu Li, Tingyi Zong. The study on MTT reduction method of testing NK cell activity. China Immunology Journal. 1996, 1(6): 356-358

5. Qian Wang. Modern medical experiment method. People's Health Publishing House. 1998, 482-483

6. Principle of new drug research in pre-clinic issued by Ministry of Health, People's Republic of China. 1993, 7: 141

7. principle of new drug research in pre-clinic issued by Ministry of Health, People's Republic of China. 1993, 7: 132-133

8. Principle of new drug research in pre-clinic issued by Ministry of Health, People's Republic of China. 1993, 7: 128-129

9. Yuanpei Zhang, Huaide Su. Phamalogical experiment (second edition). People's Health Publishing House. 1998, 137-138

10. Jiatai Li, clinical pharmacology(second edition). People's Health Publishing House. 1998, 1338-1339.

## EXAMPLE 1

Delivery of peptides through genetically engineered *Lactobacillus* bacterial species

The following is provided as one exemplary method to deliver the peptides used in this invention to a host as described above. A DNA sequence that encodes the peptide is synthesized by chemical means and this DNA sequence is inserted into an expression vector using standard techniques of genetic engineering familiar to those skilled in the art. The expression vector selected contains a constitutive promoter functional in *Lactobacilli*, a multiple cloning site for the introduction of DNA sequences in a specific 5' to 3' orientation as well as a selectable marker gene that confers resistance to an antibiotic (to aid in cloning procedures) and may comprise other sequences to assist in the production and/or secretion of the peptides, such as signal peptide sequences. An example of such a vector is provided by US Patent No. 5,592,908, to Pavla. . Briefly, this patent discusses several known promoters that function in *Lactobacillus* species, as well as a method for discovering novel promoters in said bacteria, any of which may be operably linked to a nucleic acid encoding a peptide used in the present invention to express the peptide in *Lactobacilli*. A nucleic acid encoding a signal peptide, such as peptides comprising of 16 to 35 mostly hydrophobic amino acids that are active in *Lactobacillus lactis* described in US Patent No. 5,529,908, cited above is interposed between the promoter and the nucleic acid encoding the peptide used in the present invention such that the the nucleic acid encoding the signal peptide is in frame with the nucleic acid encoding the peptide.

In addition to the coding sequence of the peptide, the DNA sequence synthesized may comprise sequences to aid in the ligation and cloning of said DNA into the expression vector. For example, restriction enzyme recognition sites that correspond to ones found in the multiple cloning site of the vector can be incorporated into the synthesized DNA at the 5' and 3' ends of the sequence, so that the sequence can be cloned in proper orientation within the vector. Both the vector and the synthesized DNA are digested with the particular restriction enzymes, then purified. Ligation reactions with the vector and the synthesized DNA are followed by transformation into a suitable strain of *E. Coli*. The transformed bacteria are plated on media containing the antibiotic to which the vector confers resistance. A colony of transformed bacteria is selected for growth cultures and plasmid preparation procedures; the presence of the synthesized DNA in the correct orientation is confirmed.

This expression vector is then transformed into a bacterial host cell of a *Lactobacillus* species, such as *L. acidophilus*. Transformed cells are selected for by virtue of the selectable marker found within the vector sequence and the secretion of the peptide may be verified by performing a western blot, performing gel electrophoresis of peptides present in the growth medium or other standard techniques. A transformed colony of bacteria is chosen and used to prepare large-scale cultures of the genetically engineered bacteria. A culture of the genetically engineered bacteria expressing the desired peptide is grown up and at least a portion thereof is administered to the alimentary canal, vagina, trachea or other area of the host organism in which the bacteria are able to replicate. If desired, the bacterial cultures can be treated in a variety of ways to produce a supplement for enteric consumption by the host. These treatments include lyophilization or other methods of preserving the bacteria, in addition to combining the bacteria with carrier agents, such as solutions, solvents, dispersion media, delay agents, emulsions and the like. The use of these agents to prepare supplements is well known in the art. For example, the bacteria can be used to make cultured milk products or other foodstuffs for human consumption, such that the organism expressing the peptide colonizes the gut of the host organism. A number of different methods for incorporating specific strains of lactic acid bacteria into foodstuffs such as yogurt, kimchee, cheese and butter are disclosed in US Patent No. 6,036,952, to Oh. Upon consuming the bacteria through one of any number of routes, the engineered organisms can colonize the gut and allow the presentation and/or absorption of the peptides of this invention via the mucosal layer of the gut.

## EXAMPLE 2

### Delivery of peptides through a genetically engineered form of *Bacillus subtilis*

The following is provided as another exemplary method to deliver the peptide useful in this invention to a host as described above. A DNA sequence that encodes the peptide is synthesized by chemical means and this DNA sequence is inserted into an expression vector via techniques of genetic engineering, all techniques being known in the art. The expression vector selected comprises a shuttle vector, such as pTZ18R (Pharmacia, Piscataway, NJ), capable of being propagated in both *E. Coli* and *B. Subtilis* and containing an antibiotic resistance gene for selecting colonies of transformed bacteria. This vector can contain a constitutive promoter active in *B. subtilis,* such as a promoter derived from the Sac B gene of *B. subtilis* as well as a nucleotide sequence encoding a signal or leader peptide active in *B. subtilis* that directs efficient export of expressed heterologous proteins from the bacterial cell. An example of such a vector is disclosed in US Patent No. 6,268,169, to Fahnestock. Briefly, as detailed above, the DNA encoding a peptide will be synthesized with restriction enzymes sites and/or other sequences to facilitate cloning of the DNA through techniques familiar to those with skill in the art. After transformation into *E. Coli.,* plating, selection and propagation of the plasmid to create a plasmid stock, the plasmid is then be transformed into *B. subtilis* and transformants are selected by virtue of resistance to an antibiotic in the plating media.

Peptide production in and secretion from the genetically engineered *B. subtilis* is verified using techniques well known to those with skill in the art, such as radiolabeling of peptides for autoradiographic detection after SDS-PAGE anaylsis or Western blotting.

A culture of genetically engineered bacteria is grown up and at least a portion thereof is administered to the alimentary canal, vagina, trachea or other area of the host organism in which the bacteria are able to replicate.

## EXAMPLE 3

### Delivery of peptides through genetically engineered *Saccharomyces* yeast species

The following is provided as another exemplary method to deliver the peptide used in this invention to a host as described above. A DNA sequence that encodes the peptide is synthesized by chemical means and this DNA sequence is inserted into an expression vector via techniques of genetic engineering, all techniques being known in the art. The expression vector selected comprises a stably maintained yeast protein expression vector, comprising a constitutive yeast promoter such as pADH1, sites for replication of the vector in both yeast and *E. Coli,* a gene or genes that confer prototrophy to an auxotrophic yeast mutant for selection purposes, a multiple cloning site (MCS) and, if desired, sequences that code for a signal peptide. Vectors such as this are commercially available and well known in the art or can be readily constructed using standard techniques After insertion of the synthesized DNA into the yeast vector, transformation into *E. Coli,* plating of transformed *E. Coli* onto selective media, selection of a transformed bacterial colony and preparation of plasmid DNA from a growth culture of bacteria from said colony, the vector is transformed into *Saccharomyces cerevisiae* via well-known techniques such as lithium acetate transformation or electroporation. The strain of *Soccharomyces cerevisiae* selected for transformation is a mutant auxotrophic strain that will require a gene on the plasmid in order to grow on minimal media plates. Transformed yeast colonies are isolated by plating the yeast on growth media lacking the gene provided on the vector. Only those yeast that have received the vector and its selective gene and are expressing that gene product will be able to grow into colonies on the minimal media. Verification of peptide secretion can be obtained by performing a Western blot, performing gel electrophoresis of peptides present in the growth medium or other standard techniques.

A transformed colony of yeast is chosen and used to prepare large scale cultures. A culture of the genetically engineered yeast expressing the desired peptide is grown up and at least a portion thereof is administered to the alimentary canal, vagina, trachea or other area of the host organism in which the bacteria are able to replicate. If desired, the yeast cultures can be treated in a variety of ways to produce a supplement for enteric consumption by the host. These treatments include lyophilization or other methods of preserving yeast, in addition to combining the bacteria with carrier agents, such as solutions, solvents, dispersion media, delay agents, emulsions and the like. The use of these agents to prepare supplements is well known in the art. The transformed yeast may also be used in the creation of food products, such as fermented milk products like yogurt and kefir, by techniques known to those skilled in the art. As with live lactic acid bacterial cultures in these foodstuffs, the transformed yeast colonize the gut at least transiently and serve to present peptides to the host via the gut lumen.

**Claims**

1. A pharmaceutical composition comprising a peptide consisting of an amino acid sequence YSL (SEQ ID NO. 16).

2. A method of making a pharmaceutical composition comprising providing a peptide as defined in claim 1; and admixing said peptide with a pharmaceutically acceptable carrier.

3. A peptide as defined in claim 1 for use in the treatment of disease.

4. Use of a peptide as defined in claim 1 in the manufacture of a medicament for modulating at least one of the following conditions: immune activity and the growth of cancer.

5. A peptide as defined in claim 1 for use in modulating at least one of the following conditions: immune activity and the growth of cancer.

6. The use according to claim 4 or 5, wherein said cancer is liver cancer or growth of melanoma.

**Patentansprüche**

1. Pharmazeutische Zusammensetzung enthaltend ein Peptid bestehend aus einer Aminosäuresequenz YSL (SEQ ID NO. 16).

2. Verfahren zur Herstellung einer pharmazeutischen Zusammensetzung umfassend Bereitstellen eines Peptids wie in Anspruch 1 definiert und Mischen des Peptids mit einem pharmazeutisch annehmbaren Träger.

3. Peptid wie in Anspruch 1 definiert zur Verwendung bei der Behandlung einer Krankheit.

4. Verwendung eines Peptids wie in Anspruch 1 definiert zur Herstellung eines Medikaments zur Modulierung von wenigstens einem der folgenden Zustände: Immunaktivität und Krebswachstum.

5. Peptid wie in Anspruch 1 definiert zur Verwendung bei der Modulierung von wenigstens einem der folgenden Zustände: Immunaktivität und Krebswachstum.

6. Die Verwendung nach einem der Ansprüche 4 oder 5, wobei der Krebs Leberkrebs oder Melanomwachstum ist.

**Revendications**

1. Composition pharmaceutique comprenant un peptide consistant en une séquence d'aminoacides YSL (SEQ ID N° 16).

2. Procédé pour la préparation d'une composition pharmaceutique, comprenant les étapes consistant à fournir un peptide tel que défini dans la revendication 1, et à mélanger ledit peptide à un support pharmaceutiquement acceptable.

3. Peptide suivant la revendication 1, destiné à être utilisé dans le traitement d'une maladie.

4. Utilisation d'un peptide suivant la revendication 1 dans la production d'un médicament destiné à la modulation d'au moins une des affections suivantes : une activité immunitaire et la croissance d'un cancer.

5. Peptide suivant la revendication 1, destiné à être utilisé dans la modulation d'au moins une des affections suivantes : une activité immunitaire et la croissance d'un cancer.

6. Utilisation suivant la revendication 4 ou 5, dans laquelle ledit cancer est le cancer du foie ou la croissance d'un mélanome.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6191113 B
- US 6184208 B
- US 6100388 A, Casas
- US 5728571 A, Bellini
- US 6391585 A, Jang
- WO 9535389 A, Leer
- US 6207456 B

- US 6245427 B
- US 5592908 A, Pavla
- US 5529908 A
- US 6036952 A, Oh
- US 6268169 B, Fahnestock
- US 09904492 B

**Non-patent literature cited in the description**

- **SQUIRE et al.** *J. Biol. Chem,* 1991, vol. 266 (33), 22366-22363
- **FURKA et al.** *J. Comb. Chem.,* 2000, vol. 2, 220-223
- Principles of Pre-clinical Research of New Drugs. People's Republic of China, 1993, vol. 7, 137-143
- Principles of Pre-clinical Research of New Drugs. People's Republic of China, 1993, vol. 7, 128-129
- **YUANPEI ZHANG ; HUAIDE SU.** Pharmacological experiment. People's Health Publishing House, 1998, 137-138
- **SHUYUN XU ; RULIAN BIAN ; XIU CHEN.** Methodology of pharmacological experiment. People's Health Publishing House, 1991, 1221-1234
- Principles of Pre-clinical Research of New Drugs. People's Republic of China, 1993, vol. 7, 140
- **JINSHENG HE ; RUIZHU LI ; TINGYI ZONG.** The study on MTT reduction method of testing NK cell activity. *China Immunology Journal,* 1996, vol. 1 (6), 356-358
- **YAOQIN YANG ; HUCHUAN YANG ; HUIHONG TAO et al.** The synergic effect of Tween-80 on the antitumor of hyperthermia-Experimental studies of mouse melanoma. *Cancer Research on Prevention and Treatment,* 1999, vol. 26 (4), 8-12
- **JIAN FU ; JIE ZHENG ; WEIGANG FANG et al.** Interleukin-12 gene transfection into murine B16 melanoma cells suppresses tumorigenicity and decreases metastatic potential. *National Medical Journal of China,* 1998, vol. 78 (8), 627-629
- Modem medical experiment method. **QIAN WANG.** People's Health Publishing House. 1998, 482-483
- **QICHAO PAN ; BIN XU.** Cancer pharmacology and chemotherapy. Henan medical university Publishing House, 2000, 66-69

- **YUANPEI ZHANG ; HUAIDE SU.** Pharmacological experiment. People's Health Publishing House, 1998, 131
- **MIZUNO, M. et al.** *Jpn J Cancer Res,* 1998, vol. 89, 76-80
- **MILLER, A.D. et al.** *Methods Enzymol,* 1993, vol. 217, 581-599
- **GOLDMAN, M.J. et al.** *Hum Gene Ther,* 1997, vol. 8, 2261-2268
- **GAO, X. ; HUANG, L.** *Gene Ther,* 1995, vol. 2, 710-722
- Principles of Pre-clinical Research of New Drugs. People's Republic of China, 1993, vol. 7, 134-135
- **SHUYUN XU ; RULIAN BIAN ; XIU CHEN.** Methodology of pharmacological experiment. People's Health Publishing House, 1991, 1221-1234
- Principle of new drug research in pre-clinic issued by Ministry of Health. People's Republic of China, 1993, vol. 7, 140
- **JINSHENG HE ; RUIZHU LI ; TINGYI ZONG.** The study on MTT reduction method of testing NK cell activity. *China Immunology Journal.,* 1996, vol. 1 (6), 356-358
- **QIAN WANG.** Modern medical experiment method. People's Health Publishing House, 1998, 482-483
- Principle of new drug research in pre-clinic issued by Ministry of Health. People's Republic of China, 1993, vol. 7, 141
- principle of new drug research in pre-clinic issued by Ministry of Health. People's Republic of China, 1993, vol. 7, 132-133
- Principle of new drug research in pre-clinic issued by Ministry of Health. People's Republic of China, 1993, vol. 7, 128-129

- **YUANPEI ZHANG ; HUAIDE SU.** Phamalogical experiment. People's Health Publishing House, 1998, 137-138

- **JIATAI LI.** clinical pharmacology. People's Health Publishing House, 1998, 1338-1339